(19) Europäisches Patentamt
European Patent Office
Office européen des brevets

(11) Veröffentlichungsnummer: **0 390 739 B1**

(12) **EUROPÄISCHE PATENTSCHRIFT**

(45) Veröffentlichungstag der Patentschrift: **09.08.95**

(51) Int. Cl.[6]: **C08K 5/3415**, C08L 57/08, C07D 207/34, C07D 403/12, C07D 403/14

(21) Anmeldenummer: **90810218.9**

(22) Anmeldetag: **19.03.90**

(54) **Substituierte Aminopyrrole als Stabilisatoren chlorhaltige Polymerisate.**

(30) Priorität: **28.03.89 CH 1117/89**

(43) Veröffentlichungstag der Anmeldung:
**03.10.90 Patentblatt 90/40**

(45) Bekanntmachung des Hinweises auf die Patenterteilung:
**09.08.95 Patentblatt 95/32**

(84) Benannte Vertragsstaaten:
**BE DE FR GB IT NL**

(56) Entgegenhaltungen:
**EP-A- 0 022 087**
**DE-A- 2 439 284**

**JOURNAL OF HETEROCYCLIC CHEMISTRY, Band 23, Nr. 5, September-Oktober 1986, Seiten 1561-1564, Provo, US; E. TOJA et al.: "Pyrrolopyridine analogs of nalidixic acid. 2. Pyrrolo[3,4-b]pyridines"**

**JOURNAL OF HETEROCYCLIC CHEMISTRY, Band 22, Nr. 1, Januar-Februar 1985, Seiten 83-88, Provo, US; S.M. BAYOMI et al.: "Synthesis of 7-oxopyrrolo(3,2-b)pyridine-6-carboxylic acid derivatives as potential antimicrobial agents"**

(73) Patentinhaber: **CIBA-GEIGY AG**
**Klybeckstrasse 141**
**CH-4002 Basel (CH)**

(72) Erfinder: **Wehner, Wolfgang, Dr.**
**Wetzbach 34**
**D-6144 Zwingenberg (DE)**
Erfinder: **Wirth, Hermann O., Dr.**
**Lessingstrasse 24**
**D-6140 Bensheim 3 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft chlorhaltige Polymerisate enthaltend Aminopyrrole, die Verwendung der Aminopyrrolverbindungen zum Stabilisieren von chlorhaltigen Polymerisaten gegen thermischen und lichtinduzierten Abbau sowie neue Aminopyrrole.

Es ist bekannt, dass chlorhaltige Polymerisate gegen den schädigenden Einfluss von Licht und Wärme, insbesondere bei der Verarbeitung zu Formteilen, geschützt werden müssen. Die Verwendung von Pyrrolen als Stabilisatoren für chlorhaltige Thermoplasten wird z.B. in EP-A-22087 beschrieben.

Die Herstellung von substituierten Aminopyrrolen wird beispielsweise in folgenden Publikationen beschrieben: E. Toja et al.; J. Heterocyclic Chem. 23, 1561 (1986); J.R. Ross et al.; J. Heterocyclic Chem. 22, 817 (1985); S.M. Bayomi et al.; J. Heterocyclic Chem. 22, 83 (1985).

Die Verwendung von Aminopyrrolen als Arzneimittel wird z.B. in US-A-3 993 650 und DE-A-2 439 284 beschrieben.

Die vorliegende Erfindung betrifft Zusammensetzungen enthaltend a) ein chlorhaltiges Polymerisat und b) mindestens eine Verbindung der Formel I,

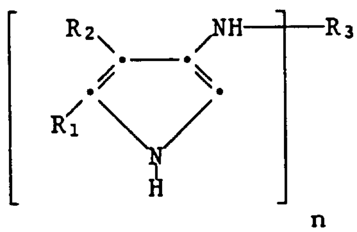

(I)

worin n 1 oder 2 ist, $R_1$ $C_1$-$C_4$-Alkyl bedeutet, $R_2$ eine Gruppe der Formel IIa, IIb, IIc oder IId darstellt,

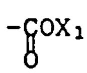 , 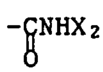 , 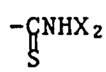 , 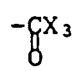 ,

(IIa) (IIb) (IIc) (IId)

$X_1$ Wasserstoff, $C_1$-$C_{20}$-Alkyl, durch ein oder zwei Sauerstoffatome oder Schwefelatome unterbrochenes oder/und durch OH substituiertes $C_3$-$C_6$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_{10}$-Alkyl, Chlor, Hydroxy, Methoxy oder/und Ethoxy substituiertes Phenyl, $C_7$-$C_{10}$-Phenylalkyl oder am Phenylrest durch $C_1$-$C_{20}$-Alkyl, Chlor, Hydroxy, Methoxy oder/und Ethoxy substituiertes $C_7$-$C_{10}$-Phenylalkyl ist, $X_2$ Phenyl oder durch 1 bis 3 Reste substituiertes Phenyl darstellt, wobei die Reste ausgewählt werden aus der Gruppe bestehend aus $C_1$-$C_4$-Alkyl, Chlor, Hydroxy, Methoxy, Ethoxy und Acetylamino, $X_3$ $C_1$-$C_8$-Alkyl oder Phenyl bedeutet, wenn n 1 ist, $R_3$ eine Gruppe der Formeln IIIa bis IIIg bedeutet,

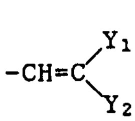 , 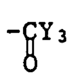 , 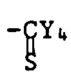 , 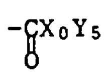 , 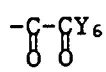 , 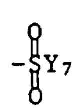 ,

(IIIa) (IIIb) (IIIc) (IIId) (IIIe) (IIIf)

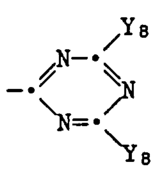 ,

(IIIg)

$X_0$ ein Sauerstoffatom oder Schwefelatom ist, $Y_1$ und $Y_2$ unabhängig voneinander -CN, Benzoyl, $C_2$-$C_4$-Alkanoyl oder $C_2$-$C_4$-Alkoxycarbonyl sind, $Y_3$ $C_1$-$C_{20}$-Alkyl, $C_3$-$C_{20}$-Alkenyl, Phenyl, durch $C_1$-$C_4$-Alkyl, Chlor, $-NO_2$, Methoxy oder/und Ethoxy substituiertes Phenyl, 2-Phenylethenyl, Di($C_1$-$C_4$-alkyl)amino, Diphenylamino, $C_1$-$C_{20}$-Alkylamino, $C_3$-$C_8$-Cycloalkylamino, Phenylamino, am Phenylring durch $C_1$-$C_4$-Alkyl, Chlor, Hydroxy, Methoxy oder/und Ethoxy substituiertes Phenylamino, Benzylamino, Benzolsulfonamido oder Toluolsulfonamido darstellt, $Y_4$ Di($C_1$-$C_4$-alkyl)amino, Diphenylamino, $C_1$-$C_8$-Alkylamino, Phenylamino, am Phenylring durch $C_1$-$C_4$-Alkyl, Chlor, Hydroxy, Methoxy oder/und Ethoxy substituiertes Phenylamino oder Benzylamino ist, $Y_5$ $C_1$-$C_{20}$-Alkyl, durch ein oder zwei Sauerstoffatome unterbrochenes $C_3$-$C_6$-Alkyl, $C_5$-$C_{12}$-Cycloalkyl, durch $C_1$-$C_4$-Alkyl substituiertes $C_5$-$C_{12}$-Cycloalkyl, Phenyl, $C_7$-$C_{10}$-Phenylalkyl oder am Phenylrest durch $C_1$-$C_{20}$-Alkyl substituiertes $C_7$-$C_{10}$-Phenylalkyl bedeutet, $Y_6$ $C_1$-$C_4$-Alkoxy, Phenylamino oder an der Phenylgruppe durch $C_1$-$C_4$-Alkyl, Chlor, Hydroxy, Methoxy oder/und Ethoxy substituiertes Phenylamino ist, $Y_7$ $C_1$-$C_4$-Alkyl, Phenyl oder durch $C_1$-$C_4$-Alkyl, Chlor, $-NO_2$, ($C_1$-$C_{12}$-Alkyl)oxycarbonyl und/oder Phenyloxycarbonyl substituiertes Phenyl darstellt, die Reste $Y_8$ unabhängig voneinander $C_1$-$C_4$-Alkoxy oder Allyloxy bedeuten, und wenn n 2 ist, $R_3$ eine Gruppe der Formel IVa, IVb, IVc oder IVd darstellt,

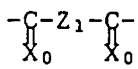
(IVa)
,
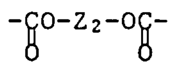
(IVb)
,
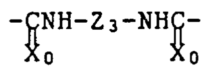
(IVc)
,
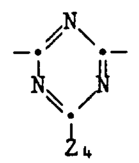
(IVd)
,

$X_0$ die oben angegebene Bedeutung besitzt, $Z_1$ eine direkte Bindung, $C_1$-$C_{12}$-Alkylen oder Phenylen bedeutet, $Z_2$ $C_2$-$C_{12}$-Alkylen oder 3-Oxapentylen ist, $Z_3$ $C_4$-$C_8$-Alkylen oder Phenylen und $Z_4$ $C_1$-$C_4$-Alkoxy oder Allyloxy darstellen.

Alkyl mit bis zu 20 C-Atomen bedeutet zum Beispiel Methyl, Ethyl, Propyl, Butyl, t-Butyl, Pentyl, Hexyl, Heptyl, Octyl, i-Octyl, i-Nonyl, Decyl, Dodecyl oder Octadecyl.

$R_1$ bedeutet vorzugsweise geradkettiges $C_1$-$C_4$-Alkyl, insbesondere Methyl.

Eine bevorzugte Bedeutung von $X_1$ ist $C_1$-$C_{18}$-Alkyl, insbesondere $C_1$-$C_{12}$-Alkyl, z.B. Methyl oder Ethyl.

$X_3$ bedeutet als Alkyl vorzugsweise Methyl oder Ethyl, insbesondere Methyl.

$Y_5$ besitzt als Alkyl vorzugsweise 1 bis 4 Kohlenstoffatome. Methyl und Ethyl sind besonders bevorzugte Bedeutungen für $Y_5$.

$C_1$-$C_4$-Alkoxy ist z.B. Methoxy, Ethoxy, Propoxy oder Butoxy.

$C_3$-$C_6$-Alkyl, welches durch 1 oder 2 Sauerstoffatome unterbrochen ist, bedeutet beispielsweise 3-Oxabutyl, 3-Oxapentyl, 3-Oxaheptyl, 3,6-Dioxaheptyl oder 3,6-Dioxaoctyl.

$C_3$-$C_6$-Alkyl, welches durch 1 oder 2 Sauerstoffatome oder Schwefelatome unterbrochen oder/und durch OH substituiert ist, kann zum Beispiel ausser den im vorangehenden Absatz angegebenen Resten auch 3-Thiabutyl, 3-Thiapentyl, 3,6-Dithiaheptyl, 3,6-Dithiaoctyl, 5-Hydroxy-3-oxapentyl, 5-Hydroxy-3-thiapentyl oder 4-Hydroxybutyl bedeuten.

$C_3$-$C_{20}$-Alkenyl ist beispielsweise Allyl, 2-Methallyl, 3-Methylbut-2-enyl, 3-Methylbut-3-enyl, Hexenyl, Decenyl, Undecenyl, Heptadecenyl oder Oleyl. Bevorzugte Bedeutungen sind Allyl, Methallyl und Oleyl.

$C_5$-$C_{12}$-Cycloalkyl, welches gegebenenfalls durch $C_1$-$C_4$-Alkyl, insbesondere Methyl, substituiert sein kann, bedeutet zum Beispiel Cyclopentyl, Cyclohexyl, Cycloheptyl, Cyclooctyl, Cyclodecyl, Cyclododecyl oder Methylcyclohexyl. Unsubstituiertes oder substituiertes $C_5$-$C_8$-Cycloalkyl, insbesondere Cyclohexyl, ist bevorzugt.

Beispiele für Phenyl, welches durch, bevorzugt 1 bis 3, definitionsgemässe Reste substituiert ist, sind o-, m- oder p-Chlorphenyl, 2,3-Dichlorphenyl, 2,4-Dichlorphenyl, 2,5-Dichlorphenyl, 2,6-Dichlorphenyl, 3,4-Dichlorphenyl, 2,4,5-Trichlorphenyl, 2,4,6-Trichlorphenyl, o-, m-oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 2-Methyl-4-tert-butylphenyl, 2-Ethylphenyl, 2,6-Diethylphenyl, 2,6-Diethyl-4-methylphenyl, 2,6-Diisopropylphenyl, 4-tert-Butylphenyl, p-Nonylphenyl, 2-Chlor-6-methylphenyl, 3-Chlor-2-methylphenyl, 3-Chlor-4-methylphenyl, 4-Chlor-2-methylphenyl, 5-Chlor-2-methylphenyl, 2,6-Dichlor-3-methylphenyl, o-, m- oder p-Methoxyphenyl, o-oder p-Ethoxyphenyl, 2,4-Dimethoxyphenyl, 2,5-Dimethoxyphenyl, 2,5-Diethoxyphenyl, 2-Methoxy-5-methylphenyl, 4-Methoxy-2-methylphenyl, 3-Chlor-4-methoxyphenyl, 3-Chlor-6-methoxyphenyl, 3-Chlor-4,6-dimethoxyphenyl, 4-Chlor-2,5-dimethoxyphenyl, o-, m- oder p-Hydroxyphenyl,

2-Hydroxy-4-methylphenyl, 3-Hydroxy-4-methylphenyl, o-, m- oder p-Acetylaminophenyl, o-, m- oder p-Nitrophenyl, p-($C_1$-$C_{12}$-Alkyl)oxycarbonylphenyl und p-Phenyloxycarbonylphenyl.

Bedeuten $Y_3$, $Y_4$ und $Y_6$ Phenylamino, welches am Phenylrest durch, bevorzugt 1 bis 3, definitionsgemässe Reste substituiert ist, so kann der substituierte Phenylrest z.B. die oben angegebenen Bedeutungen besitzen.

$C_7$-$C_{10}$-Phenylalkyl ist zum Beispiel Benzyl oder 2-Phenylethyl. Benzyl ist bevorzugt. Falls die Phenylgruppe in diesen Resten durch, bevorzugt 1 bis 3, definitionsgemässe Gruppen substituiert ist, kann sie die oben angegebenen Bedeutungen annehmen. An der Phenylgruppe durch $C_1$-$C_{20}$-Alkyl, bevorzugt $C_8$-$C_{14}$-Alkyl, substituiertes $C_7$-$C_{10}$-Phenylalkyl ist eine der bevorzugten Bedeutungen. Als Beispiel ist ferner Dodecylbenzyl zu nennen.

$C_2$-$C_4$-Alkanoyl bedeutet beispielsweise Acetyl, Propanoyl oder Butanoyl. Acetyl ist bevorzugt.

$C_2$-$C_4$-Alkoxycarbonyl ist zum Beispiel Methoxycarbonyl, Ethoxycarbonyl der Propoxycarbonyl. Methoxycarbonyl und Ethoxycarbonyl sind bevorzugt.

Di($C_1$-$C_4$-alkyl)amino bedeutet zum Beispiel Dimethylamino, Diethylamino, Dipropylamino oder Dibutylamino.

$C_1$-$C_{20}$-Alkylamino, bevorzugt $C_1$-$C_8$-Alkylamino, insbesondere $C_4$-$C_8$-Alkylamino, ist beispielsweise Butylamino, Pentylamino, Hexylamino, Heptylamino oder Octylamino.

$C_3$-$C_8$-Cycloalkylamino ist zum Beispiel Cyclopropylamino, Cyclohexylamino oder Cyclooctylamino.

Alkylen mit bis zu 12 Kohlenstoffatomen bedeutet zum Beispiel Methylen, Dimethylen, Trimethylen, Butylen, Pentamethylen, Hexamethylen, Octamethylen, Decamethylen oder Dodecamethylen. Alkylen mit bis zu 8 Kohlenstoffatomen ist bevorzugt.

Von Interesse sind Zusammensetzungen, worin

n 1 oder 2 ist, $R_1$ $C_1$-$C_4$-Alkyl bedeutet, $R_2$ eine Gruppe der Formel IIa, IIb, IIc oder IId darstellt, $X_1$ $C_1$-$C_{20}$-Alkyl, durch ein oder zwei Sauerstoffatome oder Schwefelatome unterbrochenes oder/und durch OH substituiertes $C_3$-$C_6$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_{10}$-Alkyl, Chlor, Methoxy oder/und Ethoxy substituiertes Phenyl, $C_7$-$C_{10}$-Phenylalkyl oder am Phenylrest durch $C_1$-$C_{20}$-Alkyl substituiertes $C_7$-$C_{10}$-Phenylalkyl ist, $X_2$ Phenyl oder durch 1 bis 3 Reste substituiertes Phenyl darstellt, wobei die Reste ausgewählt werden aus der Gruppe bestehend aus $C_1$-$C_4$-Alkyl, Chlor, Hydroxy, Methoxy, Ethoxy und Acetylamino, $X_3$ $C_1$-$C_8$-Alkyl oder Phenyl bedeutet, wenn n 1 ist, $R_3$ eine Gruppe der Formeln IIIa bis IIIg bedeutet, $X_0$ ein Sauerstoffatom ist, $Y_1$ und $Y_2$ unabhängig voneinander -CN, Benzoyl, $C_2$-$C_4$-Alkanoyl oder $C_2$-$C_4$-Alkoxycarbonyl sind, $Y_3$ $C_1$-$C_{20}$-Alkyl, $C_3$-$C_{20}$-Alkenyl, PHenyl, durch $C_1$-$C_4$-Alkyl, Chlor, Methoxy oder/und Ethoxy substituiertes Phenyl, 2-Phenylethenyl, Di($C_1$-$C_4$-alkyl)amino, $C_1$-$C_8$-Alkylamino, Phenylamino, Benzylamino, Benzosulfonamido oder Toluolsulfonamido darstellt, $Y_4$ Di($C_1$-$C_4$-alkyl)amino, $C_1$-$C_8$-Alkylamino, Phenylamino oder Benzylamino ist, $Y_5$ $C_1$-$C_{20}$-Alkyl, durch ein oder zwei Sauerstoffatome unterbrochenes $C_3$-$C_6$-Alkyl, $C_5$-$C_{12}$-Cycloalkyl, durch $C_1$-$C_4$-Alkyl substituiertes $C_5$-$C_{12}$-Cycloalkyl, Phenyl, $C_7$-$C_{10}$-Phenylalkyl oder am Phenylrest durch $C_1$-$C_{20}$-Alkyl substituiertes $C_7$-$C_{10}$-Phenylalkyl bedeutet, $Y_6$ $C_1$-$C_4$-Alkoxy, Phenylamino oder an der Phenylgruppe durch $C_1$-$C_4$-Alkyl, Chlor, Methoxy oder/und Ethoxy substituiertes Phenylamino ist, $Y_7$ Phenyl oder Tolyl darstellt, die Reste $Y_8$ unabhängig voneinander $C_1$-$C_4$-Alkoxy oder Allyloxy bedeuten, und wenn n 2 ist, $R_3$ eine Gruppe der Formel IVa, IVb, IVc oder IVd darstellt, $X_0$ die oben angegebene Bedeutung hat, $Z_1$ eine direkte Bindung, $C_1$-$C_{12}$-Alkylen oder Phenylen bedeutet, $Z_2$ $C_2$-$C_{12}$-Alkylen oder 3-Oxapentylen ist, $Z_3$ $C_4$-$C_8$-Alkylen oder Phenylen und $Z_4$ $C_1$-$C_4$-Alkoxy oder Allyloxy darstellen.

Bevorzugt sind auch Zusammensetzungen, worin $X_1$ $C_1$-$C_{18}$-Alkyl, durch ein oder zwei Sauerstoffatome oder Schwefelatome unterbrochenes oder/und durch OH substituiertes $C_3$-$C_6$-Alkyl, Allyl, Methallyl, Oleyl, $C_5$-$C_8$-Cycloalkyl, Phenyl, durch $C_1$-$C_{10}$-Alkyl, Chlor, Methoxy oder/und Ethoxy substituiertes Phenyl, $C_7$-$C_{10}$-Phenylalkyl oder am Phenylrest durch $C_8$-$C_{14}$-Alkyl substituiertes $C_7$-$C_{10}$-Phenylalkyl ist, $X_3$ Methyl, Ethyl oder Phenyl bedeutet, $Y_3$ $C_1$-$C_{18}$-Alkyl, Allyl, Methallyl, Oleyl, Phenyl, durch $C_1$-$C_4$-Alkyl, Chlor, Methoxy oder/und Ethoxy substituiertes Phenyl, 2-Phenylethenyl oder Di($C_1$-$C_4$-alkyl)amino ist und $Y_5$ $C_1$-$C_{18}$-Alkyl, $C_5$-$C_8$-Cycloalkyl, Phenyl, $C_7$-$C_{10}$-Phenylalkyl oder am Phenylrest durch $C_8$-$C_{14}$-Alkyl substituiertes $C_7$-$C_{10}$-Phenylalkyl darstellt.

Ebenfalls bevorzugt sind Zusammensetzungen, worin n 1 bedeutet, $R_2$ eine Gruppe der Formel IIa oder IIc ist und $R_3$ eine Gruppe der Formel IIIb, IIId oder IIIg ist.

Der Rest $R_3$ stellt vorzugsweise eine Gruppe der Formel IIIb, IIId oder IIIg dar.

Besonders bevorzugt sind Zusammensetzungen, worin n 1 bedeutet, $R_1$ Methyl ist, $R_2$ eine Gruppe der Formel IIa darstellt, $X_1$ $C_1$-$C_{12}$-Alkyl bedeutet, $R_3$ eine Gruppe der Formel IIIb, IIId oder IIIg ist, $Y_3$ $C_1$-$C_{18}$-Alkyl, Allyl, Phenyl oder durch $C_1$-$C_4$-Alkyl, Chlor, Methoxy oder/und Ethoxy substituiertes Phenyl bedeutet und $Y_5$ $C_1$-$C_{18}$-Alkyl, Cyclohexyl, Phenyl oder Benzyl darstellt.

Zusammensetzungen, worin n 1 bedeutet, $R_1$ Methyl ist, $R_2$ eine Gruppe der Formel IIa darstellt, $X_1$ $C_1$-$C_4$-Alkyl, insbesondere Methyl oder Ethyl, bedeutet, $R_3$ eine Gruppe der Formel IIId ist und $Y_5$ Methyl, Ethyl oder Phenyl bedeutet, sind eine weitere bevorzugte Ausführungsform der Erfindung.

Bevorzugt sind auch solche Zusammensetzungen, worin $R_3$ eine Gruppe der Formel IIIg ist.

Gemäss einer weiteren bevorzugten Ausführungsform bedeutet n 2.

Ferner sind solche Zusammensetzungen von Interesse, worin n 2 bedeutet und $R_3$ eine Gruppe der Formel IVa oder IVb ist.

$R_2$ ist besonders bevorzugt eine Gruppe der Formel IIa.

Bevorzugte Beispiele für Verbindungen der Formel I sind:

2-Methyl-3-methoxycarbonyl-4-ethoxycarbonylaminopyrrol,
2-Methyl-3-methoxycarbonyl-4-methoxycarbonylaminopyrrol,
2-Methyl-3-ethoxycarbonyl-4-ethoxycarbonylaminopyrrol,
2-Methyl-3-ethoxycarbonyl-4-methoxycarbonylaminopyrrol,
2-Methyl-3-methoxycarbonyl-4-benzoylaminopyrrol,
2-Methyl-3-ethoxycarbonyl-4-benzoylaminopyrrol,
2-Methyl-3-methoxycarbonyl-4-(2',4'-diallyloxy-1',3',5'-triazin-6'-yl)aminopyrrol,
2-Methyl-3-methoxycarbonyl-4-[benzyl(thiocarbonyl)]aminopyrrol,
1,4-Bis[(2'-methyl-3'-methoxycarbonylpyrrol-4'-yl)carbamoyloxy]butan,
1,6-Bis[(2'-methyl-3'-methoxycarbonylpyrrol-4'-yl)carbamoyloxy]hexan,
1,5-Bis[(2'-methyl-3'-methoxycarbonylpyrrol-4'-yl)carbamoyloxy]-3-oxapentan,
1,4-Bis[(2'-methyl-3'-methoxycarbonylpyrrol-4'-yl)carbamoyl]butan,
1,5-Bis[(2'-methyl-3'-methoxycarbonylpyrrol-4'-yl)carbamoyl]pentan,
1,6-Bis[(2'-methyl-3'-methoxycarbonylpyrrol-4'-yl)carbamoyl]hexan,
1,7-Bis[(2'-methyl-3'-methoxycarbonylpyrrol-4'-yl)carbamoyl]heptan,
1,8-Bis[(2'-methyl-3'-methoxycarbonylpyrrol-4'-yl)carbamoyl]octan,
1,4-Bis[(2'-methyl-3'-methoxycarbonylpyrrol-4'-yl)carbamoyl]benzol,
1,4-Bis[(2'-methyl-3'-methoxycarbonylpyrrol-4'-yl)aminothiocarbonyl]butan,
1,5-Bis[(2'-methyl-3'-methoxycarbonylpyrrol-4'-yl)aminothiocarbonyl]pentan,
1,6-Bis[(2'-methyl-3'-methoxycarbonylpyrrol-4'-yl)aminothiocarbonyl]hexan,
1,7-Bis[(2'-methyl-3'-methoxycarbonylpyrrol-4'-yl)aminothiocarbonyl]heptan,
1,8-Bis[(2'-methyl-3'-methoxycarbonylpyrrol-4'-yl)aminothiocarbonyl]octan,
1,4-Bis[(2'-methyl-3'-methoxycarbonylpyrrol-4'-yl)aminothiocarbonyl]benzol.

Die Verbindung 2-Methyl-3-methoxycarbonyl-4-ethoxycarbonylaminopyrrol, 2-Methyl-3-methoxycarbonyl-4-benzoylaminopyrrol, 2-Methyl-3-methoxycarbonyl-4-(2',4'-diallyloxy-1',3',5'-triazin-6'-yl)aminopyrrol, 1,4-Bis[(2'-methyl-3'-methoxycarbonylpyrrol-4'-yl)carbamoyl]butanoder 1,6-Bis-[(2'-methyl-3'-methoxycarbonylpyrrol-4'-yl)carbamoyloxy]hexan wird besonders bevorzugt in den erfindungsgemässen Zusammensetzungen eingesetzt.

Bei den chlorhaltigen Polymerisaten handelt es sich bevorzugt um Vinylchloridhomopolymere oder -copolymere. Als Comonomere für die Copolymerisate kommen z.B. in Frage: Vinylacetat, Vinylidenchlorid, Transdichlorethen, Ethylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA, ABS und MBS. Bevorzugte Substrate sind auch Mischungen der vorstehend genannten Homo- und Copolymerisate, insbesondere Vinylchlorid-Homopolymerisate, mit anderen thermoplastischen oder/und elastomeren Polymeren, insbesondere mit ABS, MBS, NBR, SAN, EVA.

Weiterhin bevorzugt sind Suspensions- und Massepolymere sowie Emulsionspolymere.

Als chlorhaltiges Polymerisat ist Polyvinylchlorid besonders bevorzugt.

Es ist vorteilhaft, die Verbindungen der Formel I zusammen mit bekannten Thermostabilisatoren einzusetzen, wie z.B. Organozinnverbindungen, Bleiverbindungen, organischen Antimonverbindungen, Me(II)-Phenolaten, insbesondere $C_7$-$C_{20}$-Alkylphenolaten, beispielsweise Nonylphenolat, oder Me(II)-Carboxylaten. Me(II) bedeutet z.B. Ba, Ca, Mg, Cd oder Zn. Bei den Carboxylaten handelt es sich bevorzugt um Salze von Carbonsäuren mit 7 bis 20 C-Atomen, beispielsweise Benzoate, Alkenoate oder Alkanoate, bevorzugt Stearate, Oleate, Laurate, Palmitate, Hydroxystearate oder 2-Ethylhexanoate. Besonders bevorzugt sind Stearate, Oleate und p-tert-Butylbenzoate. Beispiele für Organozinnverbindungen, Bleiverbindungen und organische Antimonverbindungen sind die in US-A-4 743 640 Spalte 3, Zeile 48 bis Spalte 5, Zeile 38 genannten Verbindungen.

Zusätzlich können die mit den Verbindungen der Formel I stabilisierten chlorhaltigen Polymerisate in üblichen Mengen herkömmliche PVC-Stabilisatoren enthalten, wie beispielsweise Phosphite oder Epoxyverbindungen.

Bei den Phosphiten handelt es sich bevorzugt um solche der Formeln

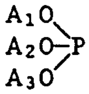, 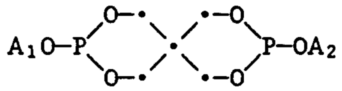, 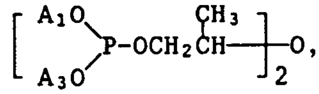, 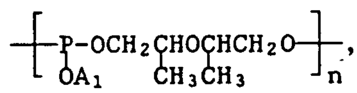,

worin $A_1$, $A_2$ und $A_3$ unabhängig voneinander $C_4$-$C_{18}$-Alkyl, $C_6$-$C_{18}$-Alkenyl, $C_5$-$C_7$-Cycloalkyl, Phenyl oder durch ein bis drei $C_1$-$C_{12}$-Alkylgruppen substituiertes Phenyl bedeuten.

Beispiele sind Trioctyl-, Tridecyl-, Tridodecyl-, Tritetradecyl-, Tristearyl-, Trioleyl-, Triphenyl-, Trikresyl-, Tris-p-nonylphenyl- und Tricyclohexylphosphit. Bevorzugt sind die Aryldialkyl- sowie die Alkyldiarylphosphite, wie z.B. Phenyldidecyl-, (2,4-Di-tert-butylphenyl)didodecyl-, (2,6-Di-tert-butylphenyl)didodecylphosphit und die Dialkyl- und Diaryl-pentaerythrit-diphosphite, wie z.B. Distearylpentaerythrit-diphosphit. Ebenfalls bevorzugt sind die Tetraphenyl- und Tetraalkyl-[dipropylenglykol-1,2]-diphosphite und die Poly-[dipropylenglykol-1,2-phenylphosphite] sowie die Poly-[dipropylenglykol-1,2-alkylphosphite].

Besonders bevorzugte organische Phosphite sind Distearyl-pentaerythritdiphosphit, Tris(nonylphenyl)-phosphit, Phenyldidecylphosphit, Tetraphenyl-[dipropylenglykol-1,2]-diphosphit und Poly-[dipropylenglykol-1,2-phenylphosphit].

Bei der Epoxyverbindung handelt es sich bevorzugt um epoxidierte Oele und epoxidierte Fettsäureester, z.B. epoxidiertes Sojabohnenöl, epoxidiertes Butyloleat und epoxidiertes Octyloleat.

Ein bevorzugter Gegenstand der Erfindung sind daher auch Zusammensetzungen enthaltend ausser der Komponente a) und einer Verbindung der Formel I mindestens ein Me(II)-Carboxylat und/oder Me(II)-Phenolat, wobei Me(II) Ba, Ca, Mg, Cd oder Zn bedeutet.

Gemäss einer weiteren Bevorzugung enthalten die erfindungsgemässen Zusammensetzungen ausser der Komponente a) und einer Verbindung der Formel I mindestens ein Me(II)-Carboxylat, wobei Me(II) Ba, Ca, Mg oder Zn bedeutet. Gemische aus Ba/Zn- oder Ca/Zn-Carboxylaten werden dabei als Costabilisatoren besonders bevorzugt.

Ebenfalls bevorzugt sind Zusammensetzungen enthaltend ausser der Komponente a) und einer Verbindung der Formel I eine Epoxyverbindung und/oder ein Phosphit und gegebenenfalls ein Me(II)-Carboxylat und/oder Me(II)-Phenolat.

Die bekannten Thermostabilisatoren (z.B. Carboxylate) können in dem zu stabilisierenden Material in einer dem Fachmann bekannten Konzentration vorliegen, wie zum Beispiel in Mengen von 0,05 bis 5 Gew.%.

Die Phosphite werden z.B. in Konzentrationen von 0,3 bis 5, vorzugsweise 0,5 bis 1 Gew.% und die Epoxyverbindungen, wie z.B. das epoxidierte Sojabohnenöl, zweckmässig in Konzentrationen von 1 bis 8, vorzugsweise 1 bis 3 Gew.% eingesetzt.

Die Verbindungen der Formel I werden beispielsweise in Mengen von 0,05 bis 5, bevorzugt 0,05 bis 1, insbesondere 0,1 bis 0,5 Gew.% in das chlorhaltige Polymerisat eingearbeitet.

Die Angabe Gew.% bezieht sich jeweils auf das zu stabilisierende Material.

Je nach dem Verwendungszweck der Polymerisate können vor oder bei der Einarbeitung der Stabilisatoren auch weitere Zusätze eingearbeitet werden, wie zum Beispiel phenolische Antioxidantien, Gleitmittel (bevorzugt Montanwachse oder Glycerinester), Fettsäureester, Paraffine, Weichmacher, Füllstoffe, Russ, Asbest, Kaolin, Talk, Glasfasern, Modifikatoren (wie etwa Schlagzäh-Zusätze), optische Aufheller, Pigmente, Lichtschutzmittel, UV-Absorber, Flammschutzmittel oder Antistatika.

Weitere mögliche Zusätze sind ferner $\beta$-Aminocrotonate, z.B. die in DE-A-804 442, DE-A-807 207 und JP-A-75/17454 beschriebenen Verbindungen, Pyrrole, z.B. die in EP-A-22 087 angegebenen Verbindungen, Aminouracile, z.B. die in EP-A-65 934 offenbarten Verbindungen, Aminothiouracile, z.B. die aus EP-A-41 479 bekannten Verbindungen, Polyole, z.B. die in DE-A-3 019 910 beschriebenen Verbindungen, $\beta$-Diketone, z.B. die in DE-A-2 600 516 angegebenen Verbindungen, oder auch Gemische aus $\beta$-Diketonen und Hydrotalciten, wie z.B. in EP-A-63 180 beschrieben.

Die Einarbeitung der Stabilisatorkomponenten in das chlorhaltige Polymerisat erfolgt am günstigsten, wie üblich, auf einem Mischwalzwerk, z.B. einem 2-Walzenstuhl bei Temperaturen zwischen 150° und 200°C. Im allgemeinen lässt sich eine genügende Homogenisierung innerhalb von 5 bis 15 Minuten erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachern gearbeitet.

Ein weiterer Gegenstand der Erfindung ist die Verwendung der Verbindungen der Formel I zum Stabilisieren von chlorhaltigen Polymerisaten gegen thermischen und lichtinduzierten Abbau.

Ebenfalls Gegenstand der Erfindung sind die neuen Verbindungen der Formel Ia,

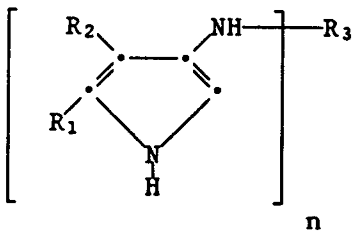 (Ia)

worin n 1 oder 2 ist, $R_1$ $C_1$-$C_4$-Alkyl bedeutet, $R_2$ eine Gruppe der Formel IIa, IIb, IIc oder IId darstellt,

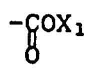 , 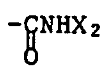 , 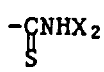 , 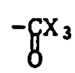 ,

(IIa) (IIb) (IIc) (IId)

$X_1$ Wasserstoff, $C_1$-$C_{20}$-Alkyl, durch ein oder zwei Sauerstoffatome oder Schwefelatome unterbrochenes oder/und durch OH substituiertes $C_3$-$C_6$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_{10}$-Alkyl, Chlor, Hydroxy, Methoxy oder/und Ethoxy substituiertes Phenyl, $C_7$-$C_{10}$-Phenylalkyl oder am Phenylrest durch $C_1$-$C_{20}$-Alkyl, Chlor, Hydroxy, Methoxy oder/und Ethoxy substituiertes $C_7$-$C_{10}$-Phenylalkyl ist, $X_2$ Phenyl oder durch 1 bis 3 Reste substituiertes Phenyl darstellt, wobei die Reste ausgewählt werden aus der Gruppe bestehend aus $C_1$-$C_4$-Alkyl, Chlor, Hydroxy, Methoxy, Ethoxy und Acetylamino, $X_3$ $C_1$-$C_8$-Alkyl oder Phenyl bedeutet, wenn n 1 ist, $R_3$ eine Gruppe der Formeln IIIb bis IIIg bedeutet,

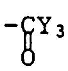 , 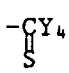 , 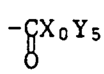 , 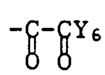 , 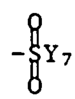 , 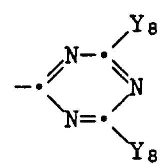 ,

(IIIb) (IIIc) (IIId) (IIIe) (IIIf) (IIIg)

$X_0$ ein Sauerstoffatom oder Schwefelatom ist, $Y_3$ $C_4$-$C_{20}$-Alkyl, $C_3$-$C_{20}$-Alkenyl, Phenyl, durch $C_1$-$C_4$-Alkyl, Chlor, $-NO_2$, Methoxy oder/und Ethoxy substituiertes Phenyl, 2-Phenylethenyl, Di($C_1$-$C_4$-alkyl)amino, Diphenylamino, $C_1$-$C_{20}$-Alkylamino, $C_3$-$C_8$-Cycloalkylamino, Phenylamino, am Phenylring durch $C_1$-$C_4$-Alkyl, Chlor, Hydroxy, Methoxy oder/und Ethoxy substituiertes Phenylamino, Benzylamino, Benzolsulfonamido oder Toluolsulfonamido darstellt, $Y_4$ Di($C_1$-$C_4$-alkyl)amino, Diphenylamino, $C_1$-$C_8$-Alkylamino, Phenylamino, am Phenylring durch $C_1$-$C_4$-Alkyl, Chlor, Hydroxy, Methoxy oder/und Ethoxy substituiertes Phenylamino oder Benzylamino ist, $Y_5$ $C_1$-$C_{20}$-Alkyl, durch ein oder zwei Sauerstoffatome unterbrochenes $C_3$-$C_6$-Alkyl, $C_5$-$C_{12}$-Cycloalkyl, durch $C_1$-$C_4$-Alkyl substituiertes $C_5$-$C_{12}$-Cycloalkyl, Phenyl oder am Phenylrest durch $C_1$-$C_{20}$-Alkyl substituiertes $C_7$-$C_{10}$-Phenylalkyl bedeutet, $Y_6$ $C_1$-$C_4$-Alkoxy, Phenylamino oder an der Phenylgruppe durch $C_1$-$C_4$-Alkyl, Chlor, Hydroxy, Methoxy oder/und Ethoxy substituiertes Phenylamino ist, $Y_7$ $C_1$-$C_4$-Alkyl, Phenyl oder durch $C_1$-$C_4$-Alkyl, Chlor, $-NO_2$, ($C_1$-$C_{12}$-Alkyl)oxycarbonyl und/oder Phenyloxycarbonyl substituiertes Phenyl darstellt, die Reste $Y_8$ unabhängig voneinander $C_1$-$C_4$-Alkoxy oder Allyloxy bedeuten, und wenn n 2 ist, $R_3$ eine Gruppe der Formel IVa, IVb, IVc oder IVd darstellt,

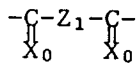 , 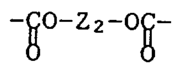 , 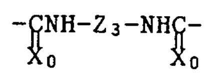 , 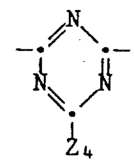 ,

(IVa) (IVb) (IVc) (IVd)

$X_0$ die oben angegebene Bedeutung besitzt, $Z_1$ eine direkte Bindung, $C_1$-$C_{12}$-Alkylen oder Phenylen bedeutet, $Z_2$ $C_2$-$C_{12}$-Alkylen oder 3-Oxapentylen ist, $Z_3$ $C_4$-$C_8$-Alkylen oder Phenylen und $Z_4$ $C_1$-$C_4$-Alkoxy oder Allyloxy darstellen, mit der Maßgabe, daß $R_3$ nicht für Benzoyl, $C_4$-Alkenoyl, $C_1$-$C_4$-Alkylsulfonyl, Phenylsulfonyl, oder Tolysulfonyl steht, wenn $R_2$ $C_2$-$C_4$-Alkanoyl oder Benzoyl bedeutet Bevorzugte Bedeutungen für die Variablen $R_1$, $R_2$, $R_3$ und n sind solche, wie sie oben für die Formel I angegeben sind.

Von Interesse sind Verbindungen der Formel Ia, worin n 1 oder 2 ist, $R_1$ $C_1$-$C_4$-Alkyl bedeutet, $R_2$ eine Gruppe der Formel IIa, IIb, IIc oder IId darstellt, $X_1$ $C_1$-$C_{20}$-Alkyl, durch ein oder zwei Sauerstoffatome oder Schwefelatome unterbrochenes oder/und durch OH substituiertes $C_3$-$C_6$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_{10}$-Alkyl, Chlor, Methoxy oder/und Ethoxy substituiertes Phenyl, $C_7$-$C_{10}$-Phenylalkyl oder am Phenylrest durch $C_1$-$C_{20}$-Alkyl substituiertes $C_7$-$C_{10}$-Phenylalkyl ist, $X_2$ Phenyl oder durch 1 bis 3 Reste substituiertes Phenyl darstellt, wobei die Reste ausgewählt werden aus der Gruppe bestehend aus $C_1$-$C_4$-Alkyl, Chlor, Hydroxy, Methoxy, Ethoxy und Acetylamino, $X_3$ $C_1$-$C_8$-Alkyl oder Phenyl bedeutet, wenn n 1 ist, $R_3$ eine Gruppe der Formeln IIIb bis IIIg bedeutet, $X_0$ ein Sauerstoffatom ist, $Y_3$ $C_4$-$C_{20}$-Alkyl, $C_3$-$C_{20}$-Alkenyl, Phenyl, durch $C_1$-$C_4$-Alkyl, Chlor, Methoxy oder/und Ethoxy substituiertes Phenyl, 2-Phenylethenyl, Di($C_1$-$C_4$-alkyl)amino, $C_1$-$C_8$-Alkylamino, Phenylamino, Benzylamino, Benzolsulfonamido oder Toluolsulfonamido darstellt, $Y_4$ Di($C_1$-$C_4$-alkyl)amino, $C_1$-$C_8$-Alkylamino, Phenylamino oder Benzylamino ist, $Y_5$ $C_1$-$C_{20}$-Alkyl, durch ein oder zwei Sauerstoffatome unterbrochenes $C_3$-$C_6$-Alkyl, $C_5$-$C_{12}$-Cycloalkyl, durch $C_1$-$C_4$-Alkyl substituiertes $C_5$-$C_{12}$-Cycloalkyl, Phenyl oder am Phenylrest durch $C_1$-$C_{20}$-Alkyl substituiertes $C_7$-$C_{10}$-Phenylalkyl bedeutet, $Y_6$ $C_1$-$C_4$-Alkoxy, Phenylamino oder an der Phenylgruppe durch $C_1$-$C_4$-Alkyl, Chlor, Methoxy oder/und Ethoxy substituiertes Phenylamino ist, $Y_7$ Phenyl oder Tolyl darstellt, die Reste $Y_8$ unabhängig voneinander $C_1$-$C_4$-Alkoxy oder Allyloxy bedeuten, und wenn n 2 ist, $R_3$ eine Gruppe der Formel IVa, IVb, IVc oder IVd darstellt, $X_0$ die oben angegebene Bedeutung hat, $Z_1$ eine direkte Bindung, $C_1$-$C_{12}$-Alkylen oder Phenylen bedeutet, $Z_2$ $C_2$-$C_{12}$-Alkylen oder 3-Oxapentylen ist, $Z_3$ $C_4$-$C_8$-Alkylen oder Phenylen und $Z_4$ $C_1$-$C_4$-Alkoxy oder Allyloxy darstellen.

Ebenfalls von Interesse sind Verbindungen der Formel Ia, worin $X_1$ $C_1$-$C_{18}$-Alkyl, durch ein oder zwei Sauerstoffatome oder Schwefelatome unterbrochenes oder/und durch OH substituiertes $C_3$-$C_6$-Alkyl, Allyl, Methallyl, Oleyl, $C_5$-$C_8$-Cycloalkyl, Phenyl, durch $C_1$-$C_{10}$-Alkyl, Chlor, Methoxy oder/und Ethoxy substituiertes Phenyl, $C_7$-$C_{10}$-Phenylalkyl oder am Phenylrest durch $C_8$-$C_{14}$-Alkyl substituiertes $C_7$-$C_{10}$-Phenylalkyl ist, $X_3$ Methyl, Ethyl oder Phenyl bedeutet, $Y_3$ $C_4$-$C_{18}$-Alkyl, Allyl, Methallyl, Oleyl, Phenyl, durch $C_1$-$C_4$-Alkyl, Chlor, Methoxy oder/und Ethoxy substituiertes Phenyl, 2-Phenylethenyl oder Di($C_1$-$C_4$-alkyl)amino ist und $Y_5$ $C_1$-$C_{18}$-Alkyl, $C_5$-$C_8$-Cycloalkyl, Phenyl oder am Phenylrest durch $C_8$-$C_{14}$-Alkyl substituiertes $C_7$-$C_{10}$-Phenylalkyl darstellt.

2-Methyl-3-methoxycarbonyl-4-ethoxycarbonylaminopyrrol, 2-Methyl-3-methoxycarbonyl-4-benzoylaminopyrrol, 2-Methyl-3-methoxycarbonyl-4-(2',4'-diallyloxy-1',3',5'-triazin-6'-yl)aminopyrrol, 1,4-Bis[(2'-methyl-3'-methoxycarbonylpyrrol-4'-yl)carbamoyl]butanoder 1,6-Bis[(2'-methyl-3'-methoxycarbonylpyrrol-4'-yl)-carbamoyloxy]hexan ist eine besonders bevorzugte Verbindung der Formel Ia.

Die Verbindungen der Formeln I bzw. Ia können in Analogie zu bekannten Verfahren hergestellt werden, beispielsweise gemäss dem unten angegebenen Schema

a) 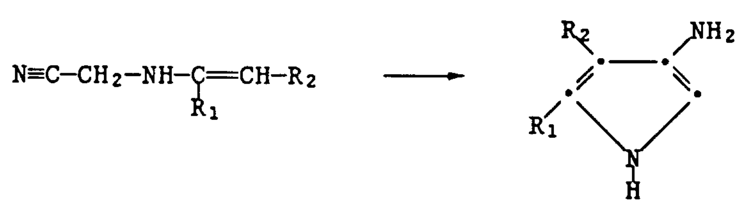

(Ib) (Ic)

b)

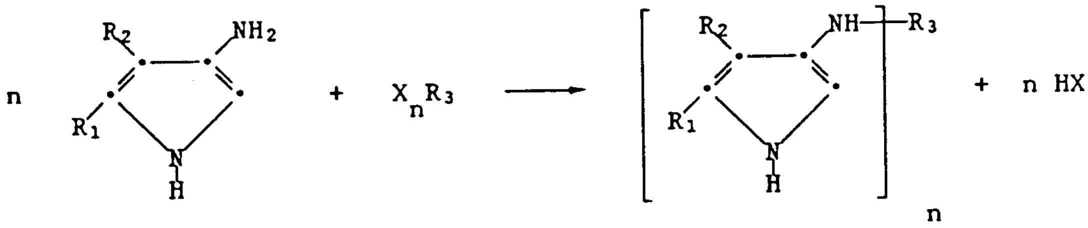

(Ic) (I)

X kann zum Beispiel Halogen, bevorzugt Chlor, bedeuten.

Die Cyclisierung a) wird zum Beispiel in einer Alkalialkoholatlösung durchgeführt. Es ist zweckmässig, die daran anschliessende Reaktion b) in situ, d.h. ohne Isolierung der Verbindung der Formel Ic vorzunehmen. Vor Durchführung der Reaktion b) ist es vorteilhaft, das Reaktionsgemisch z.B. mit Eisessig oder wässrigen Mineralsäuren zu neutralisieren. Bedeutet X Halogen, wird die Reaktion b) zweckmässig in Gegenwart eines Halogenwasserstoffakzeptors, wie z.B. eines tertiären Amins, durchgeführt.

Einige Verbindungen der Formel I bzw. Ia, worin $R_3$ eine Gruppe der Formel IIIb oder IIIc ist und $Y_3$ und $Y_4$ einen Aminorest darstellen bzw. $R_3$ eine Gruppe der Formel IVc bedeutet, können zweckmässig gemäss folgendem Schema hergestellt werden.

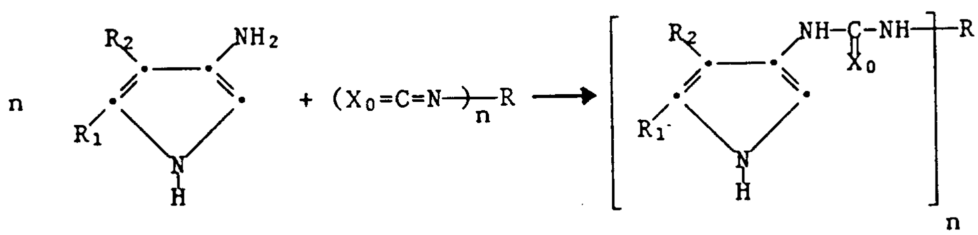

Wenn n 1 ist, bedeutet R z.B. Alkyl oder Phenyl, und wenn n 2 ist, bedeutet R z.B. Alkylen.

Die Verbindungen der Formel Ib können ebenfalls in Analogie zu bekannten Verfahren hergestellt werden, zum Beispiel durch Umsetzung von Glycinnitril oder dessen Salzen mit einer entsprechenden Ketoverbindung:

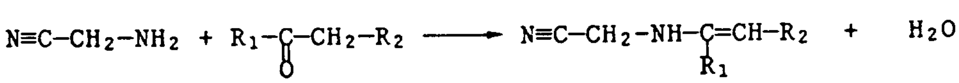

(Ib)

Werden Salze des Glycinnitrils eingesetzt, ist es vorteilhaft, die Umsetzung in Gegenwart von Säureakzeptoren, z.B. organischen oder anorganischen Basen, durchzuführen. Die Umsetzung wird bevorzugt in niederen Alkoholen bzw. Dimethylformamid oder Dimethylacetamid durchgeführt.

Verbindungen der Formel I bzw. Ia, worin $X_1$ Wasserstoff ist, werden zweckmässigerweise durch hydrierende Spaltung der entsprechenden Verbindung der Formel I bzw. Ia, worin $X_1$ Benzyl bedeutet, hergestellt.

Die folgenden Beispiele erläutern die Erfindung weiter. Teil- und Prozentangaben beziehen sich darin, soweit nichts anderes angegeben ist, auf das Gewicht.

Herstellung der Zwischenprodukte:

A) Herstellung von 2-[1'-methoxycarbonylprop-1'-en-2'-ylamino]acetonitril.

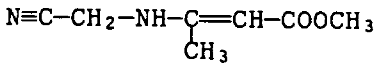

(A)

In einem 2000 ml-Dreihalskolben werden 152,6 g (1,65 Mol) Glycinnitrilhydrochlorid in 600 ml abs. Methanol unter Rühren bei 20 °C mit 167 g (1,65 Mol) Triethylamin in 50 ml abs. Methanol tropfenweise versetzt. Das Gemisch wird 10 min gerührt und anschliessend werden 174,2 g (1,5 Mol) Methylacetoacetat in 50 ml abs. Methanol zugetropft. Das Reaktionsgemisch wird 1 h am Rückfluss erhitzt. Die flüchtigen Bestandteile werden im Vakuum entfernt und der Rückstand wird in 200 ml Methanol aufgenommen. Es werden 1800 ml Eiswasser eingerührt, wobei sich ein hellgelber Niederschlag bildet, der nach Absaugen dreimal mit Eiswasser behandelt wird. Das Produkt wird bis zur Gewichtskonstanz getrocknet.

Ausbeute : 188,8 g (≙ 81,6 % der Theorie)

Schmelzpunkt: 84 °C

B) Herstellung von 2-[1'-benzoylprop-1'-en-2'ylamino]acetonitril.

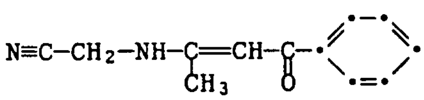

(B)

Unter Rühren werden 30,8 g (0,2 Mol) Glycinnitril-bisulfat und 32,4 g (0,2 Mol) Benzoylaceton in 400 ml Toluol vorgelegt. Es werden 33,6 g (0,4 Mol) Natriumbicarbonat portionsweise zugegeben, wobei $CO_2$-Entwicklung eintritt. Nachdem das Reaktionsgemisch 1 h am Rückfluss erhitzt worden ist, wird 1 g p-Toluolsulfonsäure hinzugegeben. Nach 4 h Reaktionszeit haben sich unter azeotropen Bedingungen 10,3 ml Wasser (berechnet: 10,8 ml) abgeschieden. Das Gemisch wird filtriert. Der Rückstand wird eingeengt und in Gegenwart von Aktivkohle aus 300 ml eines hochsiedenden Petrolether/Toluol Gemisches (1/1) umkristallisiert.

Ausbeute : 27,0 g (≙ 68 % der Theorie)

Schmelzpunkt: 105 °C

C) Herstellung von 2-[1'-t-Butoxycarbonylprop-1'-en-2'-ylamino]acetonitril.

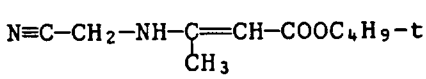

(C)

Die Verbindung wird in Analogie zu A) hergestellt. Nach Entfernung der flüchtigen Bestandteile im Vakuum wird der Rückstand mit Essigester/Wasser ausgeschüttelt, um das gebildete Triethylammoniumchlorid zu entfernen. Die Esterphase wird getrocknet und eingeengt. Der Rückstand wird aus Hexan umkristallisiert.

Ausbeute : 88 % der Theorie

Schmelzpunkt: 57 °C

D) Herstellung von 2-[1'-Acetylprop-1'-en-2'-ylamino]acetonitril.

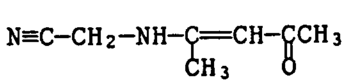

(D)

Die Verbindung wird in Analogie zu A) in Dimethylformamid als Reaktionsmedium hergestellt. Das Produkt wird aus Isopropylether umkristallisiert.

Ausbeute : 79 % der Theorie
Schmelzpunkt: 119 ° C
E-K) Die in Tabelle 1 angegebenen Verbindungen werden in Analogie zu A) in Dimethylformamid als Reaktionsmedium hergestellt.

Tabelle 1:

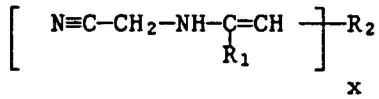

| Verbindung | x | $R_1$ | $R_2$ | Ausbeute (% der Theorie) Schmelzpunkt |
|---|---|---|---|---|
| E | 1 | $-C_3H_7$-n | $-COOCH_3$ | 60,9 % - 71°C |
| F | 1 | $-CH_3$ | —COO— 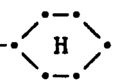 | 90 % - 79°C 1) |
| G | 1 | $-CH_3$ | $-COOC_{18}H_{37}$-n | 88 % - 79°C 1) |
| H | 1 | $-CH_3$ | $-COOC_2H_4OC_4H_9$-n | 78 % - 44°C 2) |
| I | 1 | $-CH_3$ | $-COOC_2H_4SC_2H_5$ | 62 % - 77°C 3) |
| J | 1 | $-CH_3$ | -CONH— 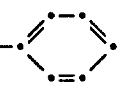 | 50 % - 110°C |
| K | 2 | $-CH_3$ | $-COOC_2H_4SC_2H_4OOC-$ | 85 % - 157°C 4) |
| L | 2 | $-CH_3$ | $-COOC_2H_4OOC-$ | 75 % - 185°C 4) |
| M | 1 | $-CH_3$ | $-COOCH_2-$ 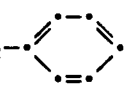 | 72 % - 97°C |

1) umkristallisiert aus Isopropylether

2) umkristallisiert aus Petrolether

3) umkristallisiert aus Diethylether

4) umkristallisiert aus Dimethylformamid/Wasser

Beispiel 1: Herstellung von 2-Methyl-3-tert-butoxycarbonyl-4-[2',2'-bis(ethoxycarbonyl)vinyl]aminopyrrol.

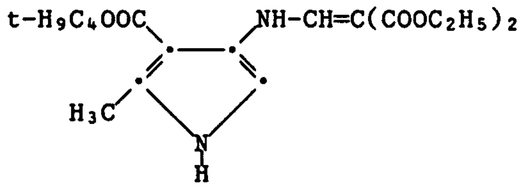

In einem 1000 ml-Dreihalskolben werden unter Rühren 43,2 g (0,24 Mol) einer 30%igen Natriummethylatlösung in 100 ml abs. Methanol vorgelegt. Anschliessend wird eine Lösung von 39,3 g (0,2 Mol) der Verbindung C in 100 ml abs. Methanol zugetropft und das erhaltene Gemisch 1 h am Rückfluss erhitzt. Dann wird das Reaktionsgemisch auf 20 °C abgekühlt und mit 14,4 g (0,24 Mol) Eisessig neutralisiert. Es wird eine Lösung von 43,3 g (0,2 Mol) Diethylethoxymethylenmalonat zugetropft und das erhaltene Gemisch wiederum 1 h am Rückfluss erhitzt. Die nahezu klare Lösung wird auf 0 °C abgekühlt. Der entstandene Niederschlag (55,5 g) wird abgesaugt und aus 800 ml Ethanol in Gegenwart von Aktivkohle umkristallisiert.

Ausbeute : 49,2 g (≙ 67,1 % der Theorie)

Schmelzpunkt: 189 °C

Beispiele 2-6: Die in Tabelle 2 angegebenen Verbindungen werden in Analogie zu Beispiel 1 hergestellt.

Tabelle 2:

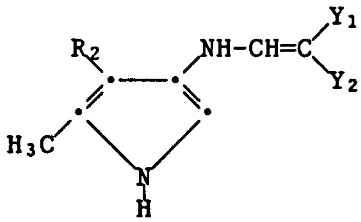

| Bsp. | eingesetztes Zwischenprodukt | $R_2$ | $Y_1$ | $Y_2$ | Ausbeute (% der Theorie) Schmelzpunkt |
|---|---|---|---|---|---|
| 2 | A | $-COOCH_3$ | $-COOC_2H_5$ | $-CN$ | 84 % - 183°C |
| 3 | B | 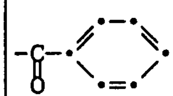 | $-COOC_2H_5$ | $-COOC_2H_5$ | 54 % - 215°C [5] |
| 4 | A | $-COOCH_3$ | $-COOC_2H_5$ | 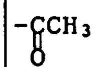 | 66 % - 180°C [6] |
| 5 | A | $-COOCH_3$ | 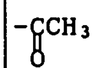 | 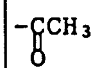 | 60 % - 192°C [6] |
| 6 | A | $-COOCH_3$ | $-COOC_2H_5$ | 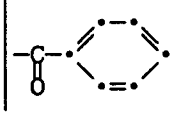 | 64 % - 171°C [6] |

[5] umkristallisiert aus Ethanol/Aktivkohle

[6] umkristallisiert aus Methanol/Aktivkohle

Beispiel 7: Herstellung von 2-Methyl-3-methoxycarbonyl-4-benzoylaminopyrrol.

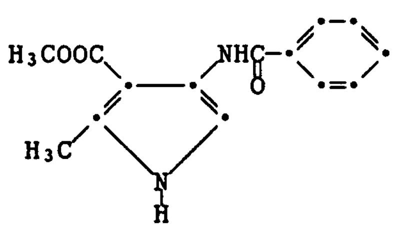

In einem 500 ml-Dreihalskolben werden unter Rühren 15,4 g (0,1 Mol) der Verbindung A in 80 ml abs. Methanol vorgelegt. Es werden 21,6 g (0,1 Mol) einer 30%igen Natriummethylatlösung zugetropft und das Reaktionsgemisch wird 1 h am Rückfluss erhitzt. Anschliessend wird das Reaktionsgemisch mit 7,2 g (0,12 Mol) Eisessig neutralisiert. Es werden 12,1 g (0,12 Mol) Triethylamin und 14,1 g (0,1 Mol) Benzoylchlorid zugetropft. Das Reaktionsgemisch wird noch 30 min bei Raumtemperatur gerührt. Nach dem Abkühlen auf 0 °C wird 1 l Eiswasser hinzugegeben. Der gebildete Niederschlag wird abgesaugt, chloridfrei gewaschen und bis zur Gewichtskonstanz getrocknet. Das Produkt wird aus Methanol/Aktivkohle umkristallisiert.
Ausbeute : 19 g (≙ 74 % der Theorie)

Schmelzpunkt: 183 °C

Beispiele 8-20: Die in Tabelle 3 angegebenen Verbindungen werden in Analogie zu Beispiel 7 hergestellt.

Tabelle 3:

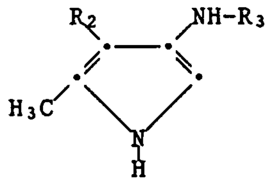

| Bsp. | eingesetztes Zwischen-produkt | $R_2$ | $R_3$ | Ausbeute (% der Theorie) Schmelzpunkt |
|---|---|---|---|---|
| 8 | A | $-COOCH_3$ | 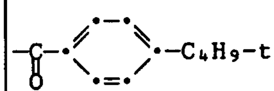 | 66 % - 220°C 6) |
| 9 | A | $-COOCH_3$ | 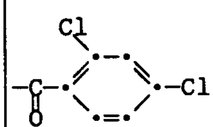 | 70,2 % - 234°C |
| 10 | A | $-COOCH_3$ | 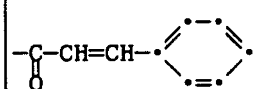 | 48,9 % - 209°C 6) |
| 11 | A | $-COOCH_3$ | 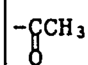 | 143°C 7) |
| 12 | A | $-COOCH_3$ | 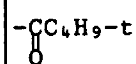 | 71 % - 164°C 7) |
| 13 | A | $-COOCH_3$ | 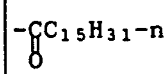 | 70 % - 78°C 6) |
| 14 | A | $-COOCH_3$ | $-COOC_2H_5$ | 80 % - 136°C 8) |
| 15 | A | $-COOCH_3$ | $-COOC_4H_9$-n | 85,7 % - 91°C |
| 16 | A | $-COOCH_3$ | $-COOC_{16}H_{33}$-n | 70 % - 94°C 6) |
| 17 | A | $-COOCH_3$ | 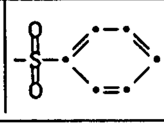 | 79 % - 223°C 8) |

Tabelle 3: (Fortsetzung)

| Bsp. | eingesetztes Zwischenprodukt | $R_2$ | $R_3$ | Ausbeute (% der Theorie) Schmelzpunkt |
|---|---|---|---|---|
| 18 | A | $-COOCH_3$ | 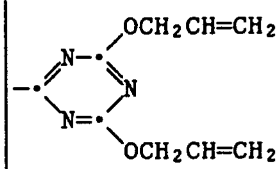 | 69 % - 170°C 8) |
| 19 | H | $-COOC_2H_4OC_4H_9-n$ | 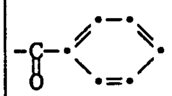 | 94°C 9) |
| 20 | I | $-COOC_2H_4SC_2H_5$ | 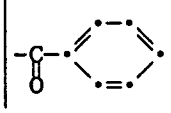 | 129°C 10) |

6) umkristallisiert aus Methanol/Aktivkohle

7) umkristallisiert aus Isopropylether/Aktivkohle

8) umkristallisiert aus Methanol/Wasser

9) Reaktion in Ethylenglykolmonobutylether an Stelle von Methanol

10) Reaktion in 3-Thiapentanol an Stelle von Methanol

Beispiel 21: Herstellung von 2-Methyl-3-methoxycarbonyl-4-phenylaminooxalylaminopyrrol.

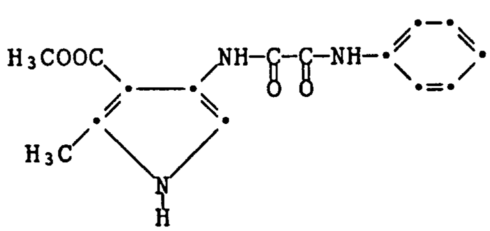

In einem 250 ml-Dreihalskolben wird eine Lösung aus 12,0 g (0,078 Mol) der Verbindung A und 16,2 g (0,09 Mol) 30%igem Natriummethylat in 60 ml abs. Methanol 30 min am Rückfluss erhitzt. Dann wird das Reaktionsgemisch mit 5,4 g (0,09 Mol) Eisessig neutralisiert. Es werden 9,1 g (0,09 Mol) Triethylamin und anschliessend 10,6 g (0,078 Mol) Oxalesterchlorid zugetropft. Das Reaktionsgemisch wird noch 1 h bei Raumtemperatur gerührt. Nachdem die flüchtigen Bestandteile im Vakuum abgezogen worden sind, wird das Reaktionsgemisch mit 37,2 g (0,4 Mol) Anilin versetzt und unter Rühren auf 160 °C erhitzt, wobei das Ethanol abdestilliert. Das überschüssige Anilin wird im Vakuum entfernt und das Reaktionsgemisch wird zweimal mit Methylenchlorid/Wasser extrahiert. Der aus der Methylenchloridphase erhaltene, dunkle Rückstand wird in 75 ml Dimethylformamid gelöst, mit Aktivkohle behandelt und in 250 ml Wasser eingerührt. Der gebildete Niderschlag wird filtriert, gewaschen und bis zur Gewichtskonstanz getrocknet.

Ausbeute : 10,2 g (≙ 42,3 % der Theorie)

Schmelzpunkt: 250 °C (Zersetzung)

Beispiel 22: Herstellung von 2-Methyl-3-cyclohexyloxycarbonyl-4-benzoylaminopyrrol.

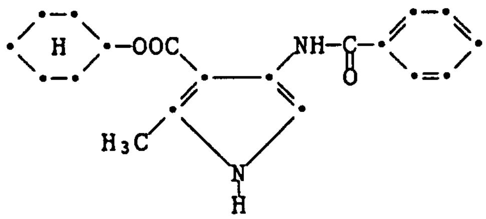

In einem 100 ml-Dreihalskolben werden unter Rühren 15,5 g (0,06 Mol) der Verbindung 7 mit 50 ml Cyclohexanol in Gegenwart von 0,5 g Titanbutylat (Katalysator) auf 160 °C erhitzt. Nach 5 h Reaktionszeit wird erneut Katalysator zugegeben. Nach ca. 15 h hat sich die quantitative Menge Methanol (2,4 ml) abgespalten. Die dunkle Reaktionslösung wird im Vakuum von flüchtigen Bestandteilen befreit, der Rückstand in Methylenchlorid aufgenommen und zweimal mit Wasser ausgeschüttelt. Nachdem die organische Phase getrocknet worden ist, wird sie in 100 ml Isopropanol unter Aktivkohlezusatz aufgenommen und unter Rühren mit 250 ml Wasser versetzt. Der gebildete Niederschlag wird aus Methanol umkristallisiert.
Ausbeute : 16,3 g (≙ 83 % der Theorie)
Schmelzpunkt: 176 °C
2-Methyl-3-cyclohexyloxycarbonyl-4-benzoylaminopyrrol kann auch in Analogie zu Beispiel 24 unter Verwendung der Vebindung F hergestellt werden.
Beispiel 23: Herstellung von 2-Methyl-3-n-octadecyloxycarbonyl-4-benzoylaminopyrrol.

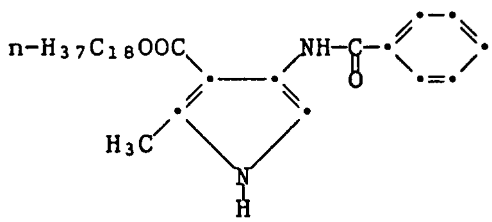

Die Verbindung wird in Analogie zu Beispiel 22 hergestellt.
Schmelzpunkt: 96 °C
2-Methyl-3-n-octadecyloxycarbonyl-4-benzoylaminopyrrol kann auch in Analogie zu Beispiel 24 unter Verwendung der Verbindung G hergestellt werden.
Beispiel 24: Herstellung von 2-Methyl-3-(5'-hydroxy-3'-thiapentyl)-4-benzoylaminopyrrol.

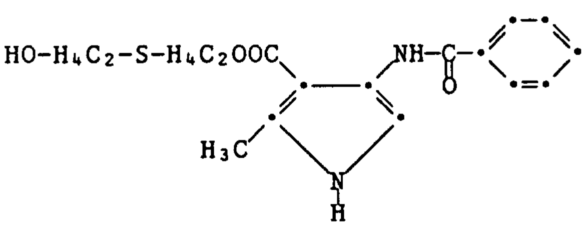

In einem 250 ml-Dreihalskolben werden 21,6 g (0,12 Mol) 30%ige Natriummethylatlösung vorgelegt. Das Methanol wird im Vakuum, möglichst quantitativ, entfernt. Es werden 70 ml Thiodiglykol zugegeben und das Gemisch wird auf 60 °C erhitzt. Im Vakuum wird das restliche Methanol entfernt. Anschliessend werden 18,3 g der Verbindung K zugegeben. Das Reaktionsgemisch wird 30 min auf 80 °C erwärmt. Dann werden 30 ml Thiodiglykol zugegeben und das Reaktionsgemisch wird auf Raumtemperatur abgekühlt. Es werden 7,2 g (0,12 Mol) Eisessig zugetropft. Nach Zugabe von 12,1 g (0,12 Mol) Triethylamin und 14,1 g (0,1 Mol) Benzoylchlorid wird das Gemisch noch 30 min bei Raumtemperatur gerührt. Anschliessend wird das Reaktionsgemisch mit Essigester/Wasser ausgeschüttelt, die organische Phase abgetrennt und mit Aktivkohle behandelt. Die flüchtigen Bestandteile werden im Vakuum entfernt. Der Rückstand wird über Kieselgel mit Methylenchlorid/Aceton (3/7) chromatographiert und das Eluat wird im Vakuum eingeengt. Anschliessend wird der Rückstand in 400 ml Methylenchlorid/Aceton (4/2) gelöst und unter Rühren mit 600 ml

Petrolether gefällt. Der gebildete Niederschlag wird bis zur Gewichtskonstanz getrocknet.
Ausbeute : 15,0 g (≙ 43 % der Theorie)
Schmelzpunkt: 114 °C
Beispiel 25: Herstellung von 2-Methyl-3-anilinocarbonyl-4-benzoylaminopyrrol.

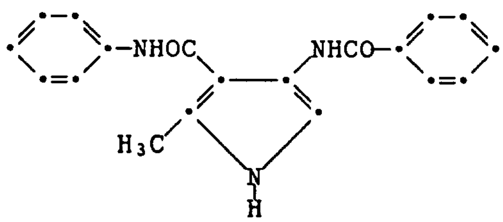

In einem 250 ml-Dreihalskolben werden 20,5 g (0,095 Mol) der Verbindung J unter Rühren in 100 ml Methanol gelöst. Es werden 18,9 g (0,105 Mol) einer 30%igen Natriummethylatlösung zugetropft und das Reaktionsgemisch wird 30 min am Rückfluss erhitzt. Nach Abkühlen auf Raumtemperatur werden 6,3 g (0,105 Mol) Eisessig zugegeben. Das Reaktionsgemisch wird 10 min gerührt und anschliessend werden 10,6 g (0,105 Mol) Triethylamin zugetropft. Nach Zugabe von 13,4 g (0,095 Mol) Benzoylchlorid wird noch 1 h bei Raumtemperatur gerührt. Das Reaktionsgemisch wird in Eiswasser gegossen und der gebildete Niederschlag salzfrei gewaschen. Das erhaltene Produkt wird bis zur Gewichtskonstanz getrocknet.
Ausbeute : 28,7 g (≙ 94,7 % der Theorie)
Schmelzpunkt: 267 °C
Beispiel 26: Herstellung von 2-Methyl-3-t-butoxycarbonyl-4-benzoylaminopyrrol.

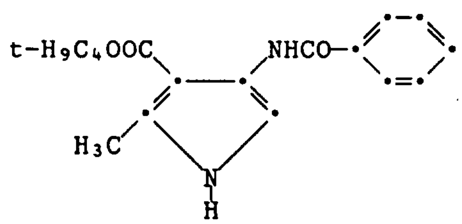

In einem 250 ml-Dreihalskolben werden 16,3 g (0,083 Mol) der Verbindung C in 100 ml Methanol vorgelegt. Unter Rühren werden 18,0 g (0,1 Mol) einer 30%igen Natriummethylatlösung zugetropft und das Reaktionsgemisch wird 30 min am Rückfluss erhitzt. Anschliessend wird mit 6,0 g (0,1 Mol) Eisessig neutralisiert und es werden 10,1 g (0,1 Mol) Triethylamin zugegeben. Nach Zutropfen von 11,7 g (0,083 Mol) Benzoylchlorid wird das Reaktionsgemisch über Nacht stehengelassen. Der gebildete Niederschlag wird abfiltriert. Das Filtrat wird eingeengt und der erhaltene Rückstand portionsweise mit Isopropylether extrahiert. Die Extrakte werden konzentriert und abgekühlt. Man erhält einen beigegefärbten Niederschlag, der bis zur Gewichtskonstanz getrocknet wird.
Ausbeute : 11,3 g (≙ 45,4 % der Theorie)
Schmelzpunkt: 170 °C
Beispiel 27: Herstellung von 2-n-Propyl-3-methoxycarbonyl-4-benzoylaminopyrrol.

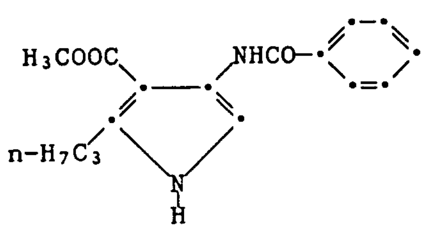

In einem 250 ml-Dreihalskolben werden 27,3 g (0,15 Mol) der Verbindung E in 80 ml Methanol gelöst und unter Rühren 30,6 g (0,17 Mol) einer 30%igen Natriummethylatlösung zugetropft. Nach 30minütigem Erhitzen am Rückfluss wird auf Raumtemperatur abgekühlt. Es werden zunächst 10,2 g (0,17 Mol) Eisessig zugegeben, nach 10 min werden dann 17,2 g (0,17 Mol) Triethylamin und anschliessend 21,1 g (0,15 Mol) Benzoylchlorid zugetropft. Das Reaktionsgemisch wird über Nacht stehengelassen. Es wird in Eiswasser eingerührt und der gebildete Niederschlag wird abfiltriert, salzfrei gewaschen und bis zur Gewichtskonstanz getrocknet.
Ausbeute : 38,6 g (≙ 95 % der Theorie)
Schmelzpunkt: 128 ° C
Beispiel 28: Herstellung von 2-Methyl-3-benzyloxycarbonyl-4-benzoylaminopyrrol.

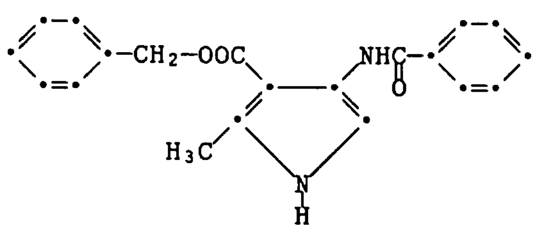

In einem 250 ml-Dreihalskolben mit Abscheider werden 38,7 g (0,15 Mol) der Verbindung aus Beispiel 7 mit 100 ml Benzylalkohol in Gegenwart von Titanbutylat 5 h unter Rühren auf 190 ° C erhitzt. Zu Beginn der Umsetzung wird zunächst 1 ml Titanbutylat und nach 3 h weitere 1 ml zugegeben. Im Verlauf der Reaktion gehen 2,3 ml (berechnet: 2,4 ml) Methanol über. Die resultierende dunkle Lösung wird im Vakuum bis zum Rückstand eingeengt. Der nicht umgesetzte Benzylalkohol wird zurückgewonnen. Der Destillationsrückstand wird in 400 ml Aceton gelöst, mit 15 ml Wasser versetzt, mit Aktivkohle behandelt und in 1,5 l Eiswasser eingerührt. Der gebildete Niederschlag wird abgesaugt und bis zur Gewichtskonstanz getrocknet.
Ausbeute: 46,1 g (≙ 91,8 % der Theorie)
Schmelzpunkt: 137 ° C
2-Methyl-3-benzyloxycarbonyl-4-benzoylaminopyrrol kann auch in Analogie zu Beispiel 24 unter Verwendung der Verbindung M hergestellt werden.
Beispiel 29: Herstellung von 2-Methyl-3-t-butoxycarbonyl-4-acetylaminopyrrol.

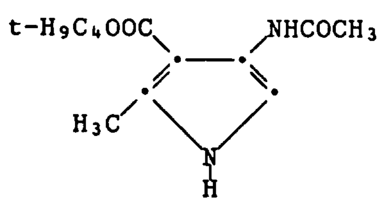

In einem 250 ml-Dreihalskolben werden 19,6 g (0,10 Mol) der Verbindung C in 100 ml Methanol gelöst. Nach Zugabe von 21,6 g (0,12 Mol) einer 30%igen Natriummethylatlösung erhitzt man 1 h am Rückfluss. Nach Abkühlen auf Raumtemperatur wird das Reaktionsgemisch mit 14,4 g (0,24 Mol) Eisessig versetzt und über Nacht stehengelassen. Es wird in Eiswasser eingerührt und der erhaltene Niederschlag wird salzfrei gewaschen und bis zur Gewichtskonstanz getrocknet.
Ausbeute : 15,4 g (≙ 68,9 % der Theorie)
Schmelzpunkt: 214 ° C
Beispiel 30: Herstellung eines Gemisches von 2-Methyl-3-t-butoxycarbonyl-4-methoxyoxalylaminopyrrol und 2-Methyl-3-t-butoxycarbonyl-4-ethoxyoxalylaminopyrrol im Molverhältnis 4:1.

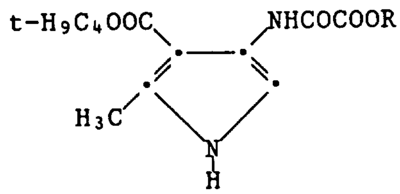

,

R = $-CH_3$ oder $C_2H_5$

In einem 250 ml-Dreihalskolben werden unter Rühren 19,6 g (0,10 Mol) der Verbindung C vorgelegt und 19,8 g (0,11 Mol) einer 30%igen Natriummethylatlösung zugetropft. Das Reaktionsgemisch wird 30 min am Rückfluss erhitzt. Nach Abkühlen auf Raumtemperatur werden 6,6 g (0,11 Mol) Eisessig und nachfolgend 11,1 g (0,11 Mol) Triethylamin und 13,7 g (0,10 Mol) Oxalsäureethylesterchlorid zugegeben. Das Reaktionsgemisch wird über Nacht stehengelassen. Es wird in Eiswasser eingerührt. Der erhaltene Niederschlag wird abfiltriert, salzfrei gewaschen und bis zur Gewichtskonstanz getrocknet.

Ausbeute : 22,6 g

Schmelzpunkt: 171 °C

Das erhaltene Gemisch kann, falls gewünscht, mit chromatographischen Methoden aufgetrennt werden.

Beispiel 31: Herstellung von 2-Methyl-3-methoxycarbonyl-4-diethylaminocarbonylaminopyrrol.

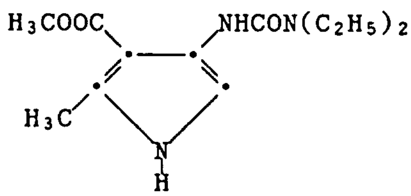

In einem 250 ml-Dreihalskolben werden 23,1 g (0,15 Mol) der Verbindung A in 80 ml Methanol gelöst. Unter Rühren werden 29,7 g (0,165 Mol) einer 30%igen Natriummethylatlösung zugetropft. Das Reaktionsgemisch wird 30 min am Rückfluss erhitzt. Nach Abkühlen auf Raumtemperatur wird mit 9,9 g (0,165 Mol) Eisessig neutralisiert. Es werden nachfolgend 16,7 g (0,165 Mol) Triethylamin und 20,3 g (0,15 Mol) N,N-Diethylcarbamidsäurechlorid zugegeben. Das Reaktionsgemisch wird über Nacht stehengelassen und eingeengt. Der Rückstand wird mit Methylenchlorid/Wasser behandelt und abfiltriert. Die beiden Phasen werden getrennt. Nach Entfernung des Lösungsmittels verbleiben aus der organischen Phase 23,3 g eines Rückstands, der mit Isopropylether extrahiert wird. Der Extrakt wird mit Aktivkohle behandelt, konzentriert und in niedrig siedenden Petrolether eingerührt. Der gebildete Niederschlag wird filtriert und getrocknet.

Schmelzpunkt: 125 °C

Beispiel 32: Herstellung von 2-Methyl-3-methoxycarbonyl-4-dimethylaminothiocarbonylaminopyrrol.

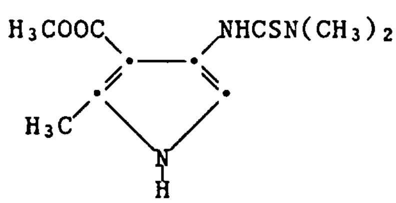

In einem 250 ml-Dreihalskolben werden 23,1 g (0,15 Mol) der Verbindung A in 80 ml Methanol vorgelegt. Nach Zugabe von 29,7 g (0,165 Mol) einer 30%igen Natriummethylatlösung wird das Reaktionsgemisch 30 min am Rückfluss erhitzt. Es wird auf Raumtemperatur abgekühlt und mit 9,9 g (0,165 Mol) Eisessig neutralisiert. Es werden nachfolgend 16,7 g (0,165 Mol) Triethylamin und 18,5 g (0,15 Mol) N,N-Dimethylthiocarbamidsäurechlorid zugegeben. Das Reaktionsgemisch wird über Nacht stehengelassen und in Eiswasser eingerührt. Der Niederschlag wird filtriert, salzfrei gewaschen und mit Methanol extrahiert. Der verbleibende Rückstand wird erneut filtriert, gewaschen und getrocknet.

Ausbeute : 15,2 g (≙ 42 % der Theorie)

Schmelzpunkt: 226 ° C

Beispiel 33: Herstellung von 2-Methyl-3-(4'-hydroxybutoxycarbonyl)-4-benzoylaminopyrrol.

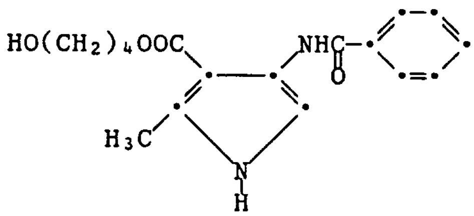

Die Verbindung wird in Analogie zu Beispiel 28 mit überschüssigem Butandiol hergestellt. Die Umsetzung wird während 5 h bei 180 ° C durchgeführt.

Ausbeute: 70 % der Theorie

Schmelzpunkt: 110 ° C

2-Methyl-3-(4'-hydroxybutoxycarbonyl)-4-benzoyl-aminopyrrol kann auch in Analogie zu Beispiel 24 unter Verwendung der Verbindung L hergestellt werden.

Beispiel 34: Herstellung von 2-Methyl-3-methoxycarbonyl-4-[2',2'-bis-(ethoxycarbonyl)vinyl]aminopyrrol.

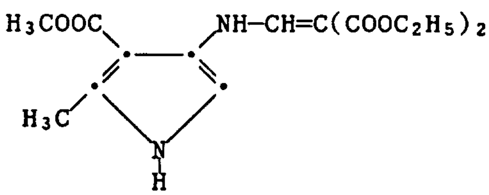

Das Produkt wird in Analogie zu Beispiel 1 unter Verwendung der Verbindung A hergestellt.

Ausbeute: 71 % der Theorie

Schmelzpunkt: 190-192 ° C (nach Umkristallisation aus Ethanol/Petrolether)

Beispiel 35: Herstellung von 2-Methyl-3-carboxy-4-benzoylaminopyrrol.

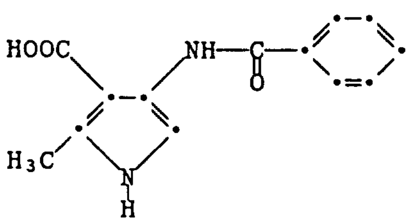

In 150 ml Dimethylacetamid werden 25,8 g (0,01 Mol) der Verbindung aus Beispiel 28 bei Raumtemperatur unter Rühren gelöst. Nach Zugabe von 5 g Pd-haltiger Kohle (5 % Pd auf Aktivkohle) wird die Hydrierung bei einem Ueberdruck von 4 bar durchgeführt. Nach 70 min ist die Wasserstoffaufnahme beendet. Der Katalysator wird abfiltriert und das Reaktionsgemisch wird im Vakuum bis zum Rückstand eingeengt. Anschliessend wird der Rückstand in 300 ml Methanol gelöst. Nach Aktivkohlebehandlung wird die Lösung in Eiswasser eingerührt. Der erhaltene Niederschlag wird abfiltriert und bis zur Gewichtskonstanz getrocknet.

Ausbeute: 11,1 g (≙ 55,5 % der Theorie)

Schmelzpunkt: 237 ° C

Beispiel 36: Herstellung von 2-Methyl-3-methoxycarbonyl-4-(n-octylthio)carbonylaminopyrrol.

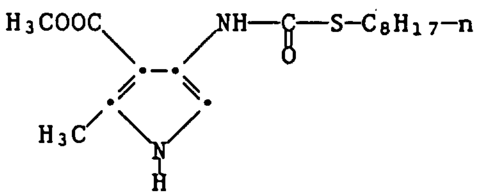

Die Verbindung wird in Analogie zu Beispiel 7 hergestellt. Als Acylierungsreagenz werden 25,1 g (0,12 Mol) n-Octylthiochlorformiat verwendet. Nach Ablauf der Reaktion wird das Reaktionsgemisch bis zum Rückstand eingeengt und dieser in Essigester/Wasser gelöst. Das erhaltene Zweiphasensystem wird zweimal mit Wasser ausgeschüttelt. Die organische Phase wird getrocknet und bis zum Rückstand eingeengt, der anschliessend aus 100 ml Methanol umkristallisiert wird. Das Produkt liegt als Hemihydrat vor.
Ausbeute: 23,6 g (≙ 58,6 % der Theorie)
Schmelzpunkt: 81 °C
Beispiel 37: Herstellung von 2-Methyl-3-methoxycarbonyl-4-anilinothiocarbonylaminopyrrol.

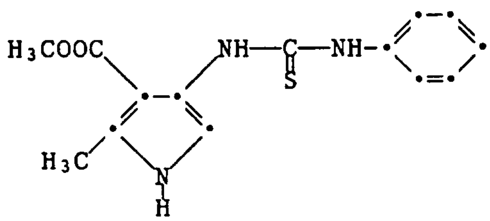

Die Herstellung erfolgt in Analogie zu Beispiel 7. Als Acylierungsreagenz werden 25,4 g (0,19 Mol) Phenylsenföl verwendet. Nach Ablauf der Reaktion wird Eiswasser zum Reaktionsgemisch gegeben. Der gebildete Niederschlag wird getrocknet und aus Aceton/Aktivkohle umkristallisiert. Das Produkt liegt als Hydrat vor (1/3 Mol $H_2O$ pro Mol Pyrrol).
Ausbeute: 57,2 % der Theorie
Schmelzpunkt: 200 °C
Beispiel 38: Herstellung von 2-Methyl-3-methoxycarbonyl-4-n-octylaminothiocarbonylaminopyrrol.

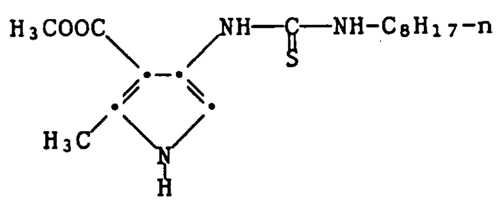

Herstellung und Aufarbeitung erfolgen in Analogie zu Beispiel 37. Als Acylierungsreagenz werden 20,5 g (0,12 Mol) Octylsenföl verwendet. Das Produkt liegt nach Umkristallisation aus Methanol/Aktivkohle als Monohydrat vor.
Ausbeute: 41,8 %
Schmelzpunkt: 103 °C
Beispiele 39-44: Die in Tabelle 4 angegebenen Verbindungen werden in Analogie zu Beispiel 21 hergestellt.

Tabelle 4:

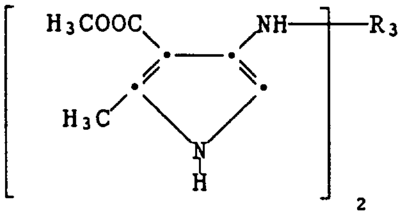

| Bsp. | eingesetztes Zwischen-produkt | $R_3$ | Ausbeute (% der Theorie) Schmelzpunkt |
|---|---|---|---|
| 39 | A | 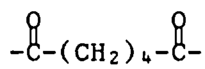 | 53 % - 270°C |
| 40 | A | 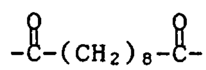 | 71 % - 205°C [11] |
| 41 | A | 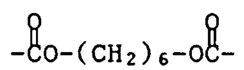 | 49 % - 191°C [11] |
| 42 | A | 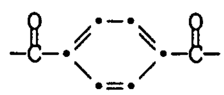 | 68 % - >200°C (Zersetzung) |
| 43 | A | 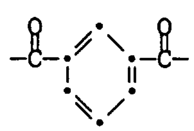 | 56 % - >200°C [11] (Zersetzung) |
| 44 | A | 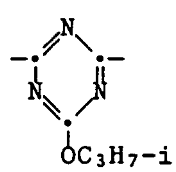 | 48 % - 190°C [8] |

8) umkristallisiert aus Methanol/Wasser

11) umkristallisiert aus Dimethylformamid/Wasser

Beispiel 45: Herstellung von 1,5-Bis[(2'-methyl-3'-methoxycarbonylpyrrol-4'-yl)carbamoyloxy]-3-oxapentan.

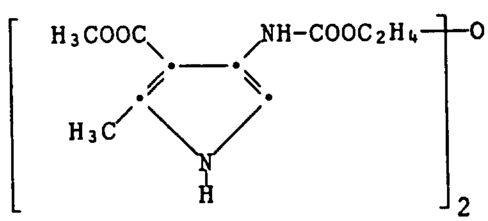

Eine Lösung von 18,5 g (0,12 Mol) der Verbindung A in 100 ml absolutem Methanol wird zusammen mit 25,9 g (0,144 Mol) Natriummethylat (30%ig in Methanol) 30 min am Rückfluss erhitzt. Nach Abkühlen auf

Raumtemperatur wird das Gemisch mit 8,64 g (0,144 Mol) Eisessig neutralisiert. Anschliessend werden nacheinander 14,6 g (0,144 Mol) Triethylamin und 13,9 g (0,06 Mol) Diglykol-bis[chlorformiat] zugetropft, wobei die Temperatur durch Kühlung auf 10 °C gehalten wird. Nach beendeter Reaktion wird auf 0 °C gekühlt. Der erhaltene Niederschlag wird abgesaugt, nacheinander mit kaltem Methanol und Wasser gewaschen und bis zur Gewichtskonstanz getrocknet. Zur weiteren Reinigung wird das Produkt aus Aceton/Dimethylacetamid (6:1) in Gegenwart von Aktivkohle umkristallisiert. Das Produkt liegt als Monohydrat vor.
Ausbeute: 26 % der Theorie
Schmelzpunkt: 206 °C

Beispiel 46: Herstellung von 1,6-Bis[(2'-methyl-3'-methoxycarbonylpyrrol-4'-yl)carbamoylamino]hexan.

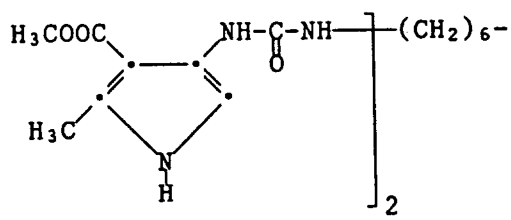

Herstellung und Aufarbeitung erfolgen in Analogie zu Beispiel 45. Als Acylierungsreagenz werden 10,1 g (0,06 Mol) Hexamethylendiisocyanat verwendet. Das Produkt liegt als Tetrahydrat vor.
Ausbeute: 30,7 % der Theorie
Schmelzpunkt: 136 °C

Beispiel 47: Die unten angegebene Trockenmischung wird auf einem Mischwalzwerk 5 min bei 180 °C gewalzt. Vom gebildeten 0,3 mm dicken Walzfell werden Folienmuster in einem Trockenschrank bei 180 °C thermisch belastet. Der Yellowness Index (YI) der Proben wird in regelmässigen Zeitabständen nach ASTM D 1925 bestimmt. Die Ergebnisse sind in den Tabellen 5 und 6a-6c aufgeführt.

Tabelle 5:

Trockenmischung: 100 Teile S-PVC (®Solvic 268 GA),
2 Teile epoxidiertes Sojabohnenöl,
0,5 Teile der angegebenen Verbindung.

| Verbindung aus Bsp. | YI-Werte nach Belastungszeit in Minuten | | | | |
|---|---|---|---|---|---|
| | 0 | 5 | 10 | 15 | 20 |
| — | 39,5 | 32,0 | 34,0 | 39,2 | 62,2 |
| 7 | 8,9 | 7,6 | 8,0 | 8,6 | 10,2 |
| 12 | 5,8 | 5,0 | 5,6 | 7,3 | 8,4 |
| 14 | 6,3 | 5,4 | 5,9 | 8,1 | 10,5 |

Tabellen 6a-6c:

Trockenmischung: 100 Teile S-PVC (®Solvic 268 GA),
3 Teile epoxidiertes Sojabohnenöl,
0,35 Teile Ca-Stearat,
0,15 Teile Zn-Stearat,
0,55 Teile Diisodecyl-phenylphosphit,
0,3 Teile der angegebenen Verbindung.

a)

| Verbindung aus Bsp. | YI-Werte nach Belastungszeit in Minuten | | | | | | |
|---|---|---|---|---|---|---|---|
| | 0 | 5 | 10 | 15 | 20 | 25 | 30 |
| — | 16,9 | 20,2 | 24,7 | 34,5 | 38,4 | 36,0 | 35,4 |
| 7 | 4,4 | 6,2 | 8,3 | 11,4 | 14,1 | 18,9 | 25,0 |
| 8 | 4,2 | 6,9 | 9,3 | 12,5 | 14,5 | 17,1 | 22,9 |
| 12 | 3,6 | 5,5 | 7,9 | 12,4 | 14,6 | 18,4 | 24,1 |
| 14 | 4,4 | 6,3 | 9,0 | 12,9 | 15,8 | 18,4 | 24,2 |

b)

| Verbindung aus Bsp. | YI-Werte nach Belastungszeit in Minuten | | | | | | |
|---|---|---|---|---|---|---|---|
| | 0 | 5 | 10 | 15 | 20 | 25 | 30 |
| — | 21,9 | 25,6 | 32,3 | 39,6 | 40,7 | 39,1 | 43,4 |
| 13 | 8,8 | 9,6 | 12,7 | 16,2 | 20,2 | 22,5 | 26,8 |
| 16 | 9,9 | 10,9 | 13,7 | 18,1 | 20,8 | 23,1 | 27,9 |

c)

| Verbindung aus Bsp. | YI-Werte nach Belastungszeit in Minuten | | | | | |
|---|---|---|---|---|---|---|
| | 0 | 5 | 10 | 15 | 20 | 25 |
| — | 19,4 | 20,3 | 28,7 | 39,0 | 39,0 | 36,1 |
| 18 | 7,8 | 8,4 | 11,6 | 15,2 | 19,6 | 24,1 |
| 19 | 8,6 | 9,5 | 11,8 | 16,2 | 20,4 | 22,9 |
| 22 | 5,8 | 6,1 | 10,4 | 14,9 | 18,3 | 20,6 |
| 24 | 7,1 | 7,6 | 9,2 | 14,4 | 18,2 | 21,7 |

Beispiel 48: Die unten angegebene Trockenmischung wird auf einem Mischwalzwerk 5 min bei 190°C gewalzt. Vom gebildeten 0,3 mm dicken Walzfell wird ein Folienstreifen im ®Mathis-Thermotester bei 180°C geprüft. Der Yellowness Index (YI) der Proben wird in regelmässigen Zeitabständen nach ASTM D 1925 bestimmt. Die Ergebnisse sind in Tabelle 7 zusammengefasst.

Tabelle 7:

Trockenmischung: 100 Teile S-PVC (®Vestolit S 6058),
5 Teile epoxidiertes Sojabohnenöl,
0,2 Teile Montansäureester-Wachs,
0,8 Teile Diisodecyl-phenylphosphit,
0,6 Teile der angegebenen Verbindung.

| Verbindung aus Bsp. | YI-Werte nach Belastungszeit in Minuten | | | | | | |
|---|---|---|---|---|---|---|---|
| | 0 | 5 | 10 | 15 | 20 | 25 | 30 |
| 18 | 9,2 | 11,5 | 12,9 | 16,1 | 22,9 | 29,5 | 34,9 |
| 37 | 5,7 | 10,4 | 12,0 | 15,7 | 19,9 | 25,3 | 30,6 |
| — | Bei der Verarbeitung der angegebenen Trockenmischung wird ein auf der Walze klebendes, dunkles Fell erhalten. | | | | | | |

Beispiel 49: Die unten angegebene Trockenmischung wird auf einem Mischwalzwerk 5 min bei 190°C gewalzt. Vom gebildeten 0,3 mm dicken Walzfell werden Folienmuster in einem Trockenschrank bei 180°C thermisch belastet. Der Yellowness Index (YI) der Proben wird in regelmässigen Zeitabständen nach ASTM D 1925 bestimmt. Die Ergebnisse sind in der Tabelle 8 aufgeführt.

Tabelle 8:

Trockenmischung: 100 Teile PVC (®Vinnol H 70 DF),
17 Teile Dioctylphthalat,
3 Teile epoxidiertes Sojabohnenöl,
0,33 Teile Zn-Oleat,
0,53 Teile Ba-p-(t-Butyl)benzoat,
0,7 Teile Diisodecyl-phenylphosphit,
0,44 Teile ®SHELL SOL A (aromatisches Kohlenwasserstoff-Gemisch),
0,2 Teile der angegebenen Verbindung.

| Verbindung aus Bsp. | YI nach Belastungszeit in Minuten | | | | | | |
|---|---|---|---|---|---|---|---|
| | 0 | 5 | 10 | 15 | 20 | 25 | 30 |
| — | 10,5 | 11,9 | 14,6 | 19,6 | 26,1 | 30,1 | 31,5 |
| 22 | 4,7 | 7,0 | 8,4 | 9,0 | 10,8 | 13,1 | 15,5 |
| 24 | 5,4 | 6,5 | 8,1 | 10,3 | 13,1 | 15,6 | 19,2 |

## Patentansprüche

1. Zusammensetzung enthaltend a) ein chlorhaltiges Polymerisat und b) mindestens eine Verbindung der Formel I,

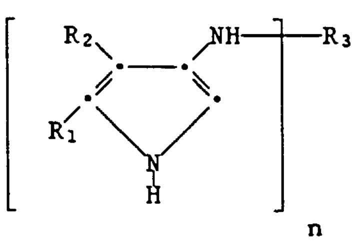

(I)

worin n 1 oder 2 ist, $R_1$ $C_1$-$C_4$-Alkyl bedeutet, $R_2$ eine Gruppe der Formel IIa, IIb, IIc oder IId darstellt,

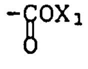 , 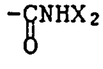 , 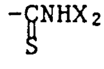 , 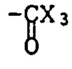 ,

(IIa) (IIb) (IIc) (IId)

$X_1$ Wasserstoff, $C_1$-$C_{20}$-Alkyl, durch ein oder zwei Sauerstoffatome oder Schwefelatome unterbrochenes oder/und durch OH substituiertes $C_3$-$C_6$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_{10}$-Alkyl, Chlor, Hydroxy, Methoxy oder/und

Ethoxy substituiertes Phenyl, $C_7$-$C_{10}$-Phenylalkyl oder am Phenylrest durch $C_1$-$C_{20}$-Alkyl, Chlor, Hydroxy, Methoxy oder/und Ethoxy substituiertes $C_7$-$C_{10}$-Phenylalkyl ist, $X_2$ Phenyl oder durch 1 bis 3 Reste substituiertes Phenyl darstellt, wobei die Reste ausgewählt werden aus der Gruppe bestehend aus $C_1$-$C_4$-Alkyl, Chlor, Hydroxy, Methoxy, Ethoxy und Acetylamino, $X_3$ $C_1$-$C_8$-Alkyl oder Phenyl bedeutet, wenn n 1 ist, $R_3$ eine Gruppe der Formeln IIIa bis IIIg bedeutet,

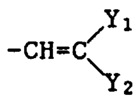 , 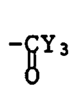 , 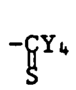 , 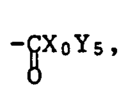 , 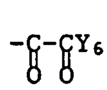 , 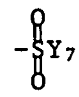 ,

(IIIa) (IIIb) (IIIc) (IIId) (IIIe) (IIIf)

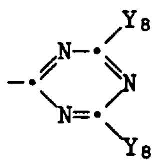 ,

(IIIg)

$X_0$ ein Sauerstoffatom oder Schwefelatom ist, $Y_1$ und $Y_2$ unabhängig voneinander -CN, Benzoyl, $C_2$-$C_4$-Alkanoyl oder $C_2$-$C_4$-Alkoxycarbonyl sind, $Y_3$ $C_1$-$C_{20}$-Alkyl, $C_3$-$C_{20}$-Alkenyl, Phenyl, durch $C_1$-$C_4$-Alkyl, Chlor, $-NO_2$, Methoxy oder/und Ethoxy substituiertes Phenyl, 2-Phenylethenyl, Di-($C_1$-$C_4$-alkyl)amino, Diphenylamino, $C_1$-$C_{20}$-Alkylamino, $C_3$-$C_8$-Cycloalkylamino, Phenylamino, am Phenylring durch $C_1$-$C_4$-Alkyl, Chlor, Hydroxy, Methoxy oder/und Ethoxy substituiertes Phenylamino, Benzylamino, Benzolsulfonamido oder Toluolsulfonamido darstellt, $Y_4$ Di($C_1$-$C_4$-alkyl)amino, Diphenylamino, $C_1$-$C_8$-Alkylamino, Phenylamino, am Phenylring durch $C_1$-$C_4$-Alkyl, Chlor, Hydroxy, Methoxy oder/und Ethoxy substituiertes Phenylamino oder Benzylamino ist, $Y_5$ $C_1$-$C_{20}$-Alkyl, durch ein oder zwei Sauerstoffatome unterbrochenes $C_3$-$C_6$-Alkyl, $C_5$-$C_{12}$-Cycloalkyl, durch $C_1$-$C_4$-Alkyl substituiertes $C_5$-$C_{12}$-Cycloalkyl, Phenyl, $C_7$-$C_{10}$-Phenylalkyl oder am Phenylrest durch $C_1$-$C_{20}$-Alkyl substituiertes $C_7$-$C_{10}$-Phenylalkyl bedeutet, $Y_6$ $C_1$-$C_4$-Alkoxy, Phenylamino oder an der Phenylgruppe durch $C_1$-$C_4$-Alkyl, Chlor, Hydroxy, Methoxy oder/und Ethoxy substituiertes Phenylamino ist, $Y_7$ $C_1$-$C_4$-Alkyl, Phenyl oder durch $C_1$-$C_4$-Alkyl, Chlor, $-NO_2$, ($C_1$-$C_{12}$-Alkyl)oxycarbonyl und/oder Phenyloxycarbonyl substituiertes Phenyl darstellt, die Reste $Y_8$ unabhängig voneinander $C_1$-$C_4$-Alkoxy oder Allyloxy bedeuten, und wenn n 2 ist, $R_3$ eine Gruppe der Formel IVa, IVb, IVc oder IVd darstellt,

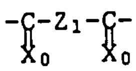 , 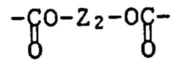 , 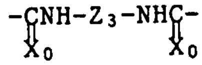 , 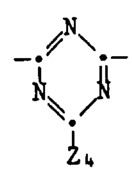 ,

(IVa) (IVb) (IVc) (IVd)

$X_0$ die oben angegebene Bedeutung besitzt,
$Z_1$ eine direkte Bindung, $C_1$-$C_{12}$-Alkylen oder Phenylen bedeutet, $Z_2$ $C_2$-$C_{12}$-Alkylen oder 3-Oxapentylen ist, $Z_3$ $C_4$-$C_8$-Alkylen oder Phenylen und $Z_4$ $C_1$-$C_4$-Alkoxy oder Allyloxy darstellen.

2. Zusammensetzung gemäss Anspruch 1, worin n 1 oder 2 ist, $R_1$ $C_1$-$C_4$-Alkyl bedeutet, $R_2$ eine Gruppe der Formel IIa, IIb, IIc oder IId darstellt, $X_1$ $C_1$-$C_{20}$-Alkyl, durch ein oder zwei Sauerstoffatome oder Schwefelatome unterbrochenes oder/und durch OH substituiertes $C_3$-$C_6$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_{10}$-Alkyl, Chlor, Methoxy oder/und Ethoxy substituiertes Phenyl, $C_7$-$C_{10}$-Phenylalkyl oder am Phenylrest durch $C_1$-$C_{20}$-Alkyl substituiertes $C_7$-$C_{10}$-Phenylalkyl ist, $X_2$ Phenyl oder durch 1 bis 3 Reste substituiertes Phenyl darstellt, wobei die Reste ausgewählt werden aus der Gruppe bestehend aus $C_1$-$C_4$-Alkyl, Chlor, Hydroxy, Methoxy, Ethoxy und Acetylamino, $X_3$ $C_1$-$C_8$-Alkyl oder Phenyl bedeutet, wenn n 1 ist, $R_3$ eine Gruppe der Formeln IIIa bis IIIg bedeutet, $X_0$ ein Sauerstoffatom ist, $Y_1$ und $Y_2$ unabhängig

voneinander -CN, Benzoyl, $C_2$-$C_4$-Alkanoyl oder $C_2$-$C_4$-Alkoxycarbonyl sind, $Y_3$ $C_1$-$C_{20}$-Alkyl, $C_3$-$C_{20}$-Alkenyl, Phenyl, durch $C_1$-$C_4$-Alkyl, Chlor, Methoxy oder/und Ethoxy substituiertes Phenyl, 2-Phenylethenyl, Di($C_1$-$C_4$-alkyl)amino, $C_1$-$C_8$-Alkylamino, Phenylamino, Benzylamino, Benzolsulfonamido oder Toluolsulfonamido darstellt, $Y_4$ Di($C_1$-$C_4$-alkyl)amino, $C_1$-$C_8$-Alkylamino, Phenylamino oder Benzylamino ist, $Y_5$ $C_1$-$C_{20}$-Alkyl, durch ein oder zwei Sauerstoffatome unterbrochenes $C_3$-$C_6$-Alkyl, $C_5$-$C_{12}$-Cycloalkyl, durch $C_1$-$C_4$-Alkyl substituiertes $C_5$-$C_{12}$-Cycloalkyl, Phenyl, $C_7$-$C_{10}$-Phenylalkyl oder am Phenylrest durch $C_1$-$C_{20}$-Alkyl substituiertes $C_7$-$C_{10}$-Phenylalkyl bedeutet, $Y_6$ $C_1$-$C_4$-Alkoxy, Phenylamino oder an der Phenylgruppe durch $C_1$-$C_4$-Alkyl, Chlor, Methoxy oder/und Ethoxy substituiertes Phenylamino ist, $Y_7$ Phenyl oder Tolyl darstellt, die Reste $Y_8$ unabhängig voneinander $C_1$-$C_4$-Alkoxy oder Allyloxy bedeuten, und wenn n 2 ist, $R_3$ eine Gruppe der Formel IVa, IVb, IVc oder IVd darstellt, $X_0$ die oben angegebene Bedeutung hat, $Z_1$ eine direkte Bindung, $C_1$-$C_{12}$-Alkylen oder Phenylen bedeutet, $Z_2$ $C_2$-$C_{12}$-Alkylen oder 3-Oxapentylen ist, $Z_3$ $C_4$-$C_8$-Alkylen oder Phenylen und $Z_4$ $C_1$-$C_4$-Alkoxy oder Allyloxy darstellen.

**3.** Zusammensetzung gemäss Anspruch 1, worin $X_1$ $C_1$-$C_{18}$-Alkyl, durch ein oder zwei Sauerstoffatome oder Schwefelatome unterbrochenes oder/und durch OH substituiertes $C_3$-$C_6$-Alkyl, Allyl, Methallyl, Oleyl, $C_5$-$C_8$-Cycloalkyl, Phenyl, durch $C_1$-$C_{10}$-Alkyl, Chlor, Methoxy oder/und Ethoxy substituiertes Phenyl, $C_7$-$C_{10}$-Phenylalkyl oder am Phenylrest durch $C_8$-$C_{14}$-Alkyl substituiertes $C_7$-$C_{10}$-Phenylalkyl ist, $X_3$ Methyl, Ethyl oder Phenyl bedeutet, $Y_3$ $C_1$-$C_{18}$-Alkyl, Allyl, Methallyl, Oleyl, Phenyl, durch $C_1$-$C_4$-Alkyl, Chlor, Methoxy oder/und Ethoxy substituiertes Phenyl, 2-Phenylethenyl oder Di($C_1$-$C_4$-alkyl)-amino ist und $Y_5$ $C_1$-$C_{18}$-Alkyl, $C_5$-$C_8$-Cycloalkyl, Phenyl, $C_7$-$C_{10}$-Phenylalkyl oder am Phenylrest durch $C_8$-$C_{14}$-Alkyl substituiertes $C_7$-$C_{10}$-Phenylalkyl darstellt.

**4.** Zusammensetzung gemäss Anspruch 1, worin $R_1$ Methyl bedeutet.

**5.** Zusammensetzung gemäss Anspruch 1, worin n 1 bedeutet, $R_2$ eine Gruppe der Formel IIa oder IIc ist und $R_3$ eine Gruppe der Formel IIIb, IIId oder IIIg ist.

**6.** Zusammensetzung gemäss Anspruch 1, worin n 1 bedeutet, $R_1$ Methyl ist, $R_2$ eine Gruppe der Formel IIa darstellt, $X_1$ $C_1$-$C_{12}$-Alkyl bedeutet, $R_3$ eine Gruppe der Formel IIIb, IIId oder IIIg ist, $Y_3$ $C_1$-$C_{18}$-Alkyl, Allyl, Phenyl oder durch $C_1$-$C_4$-Alkyl, Chlor, Methoxy oder/und Ethoxy substituiertes Phenyl bedeutet und $Y_5$ $C_1$-$C_{18}$-Alkyl, Cyclohexyl, Phenyl oder Benzyl darstellt.

**7.** Zusammensetzung gemäss Anspruch 1, worin n 2 bedeutet.

**8.** Zusammensetzung gemäss Anspruch 1, worin n 2 bedeutet und $R_3$ eine Gruppe der Formel IVa oder IVb ist.

**9.** Zusammensetzung gemäss Anspruch 1, worin $R_2$ eine Gruppe der Formel IIa ist.

**10.** Zusammensetzung gemäss Anspruch 1, worin die Verbindung der Formel I 2-Methyl-3-methoxycarbonyl-4-ethoxycarbonylaminopyrrol, 2-Methyl-3-methoxycarbonyl-4-benzoylaminopyrrol, 2-Methyl-3-methoxycarbonyl-4-(2',4'-diallyloxy-1',3',5'-triazin-6'-yl)aminopyrrol, 1,4-Bis[(2'-methyl-3'-methoxycarbonylpyrrol-4'-yl)carbamoyl]butanoder 1,6-Bis[(2'-methyl-3'-methoxycarbonylpyrrol-4'-yl)carbamoyloxy]hexan ist.

**11.** Zusammensetzung gemäss Anspruch 1, worin das chlorhaltige Polymerisat Polyvinylchlorid ist.

**12.** Zusammensetzung gemäss Anspruch 1, enthaltend zusätzlich mindestens ein Me(II)-Carboxylat und/oder Me(II)-Phenolat, wobei Me(II) Ba, Ca, Mg, Cd oder Zn bedeutet.

**13.** Zusammensetzung gemäss Anspruch 1, enthaltend zusätzlich eine Epoxyverbindung und/oder ein Phosphit.

**14.** Verwendung von Verbindungen der Formel I gemäss Anspruch 1 zum Stabilisieren eines chlorhaltigen Polymerisats gegen thermischen und lichtinduzierten Abbau.

**15.** Verbindungen der Formel Ia,

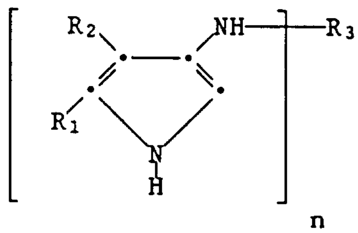

(Ia)

worin n 1 oder 2 ist, $R_1$ $C_1$-$C_1$-Alkyl bedeutet, $R_2$ eine Gruppe der Formel IIa, IIb, IIc oder IId darstellt,

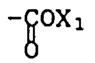 , 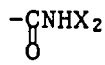 , 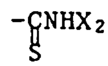 , 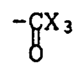 ,

(IIa) (IIb) (IIc) (IId)

$X_1$ Wasserstoff, $C_1$-$C_{20}$-Alkyl, durch ein oder zwei Sauerstoffatome oder Schwefelatome unterbrochenes oder/und durch OH substituiertes $C_3$-$C_6$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_{10}$-Alkyl, Chlor, Hydroxy, Methoxy oder/und Ethoxy substituiertes Phenyl, $C_7$-$C_{10}$-Phenylalkyl oder am Phenylrest durch $C_1$-$C_{20}$-Alkyl, Chlor, Hydroxy, Methoxy oder/und Ethoxy substituiertes $C_7$-$C_{10}$-Phenylalkyl ist, $X_2$ Phenyl oder durch 1 bis 3 Reste substituiertes Phenyl darstellt, wobei die Reste ausgewählt werden aus der Gruppe bestehend aus $C_1$-$C_4$-Alkyl, Chlor, Hydroxy, Methoxy, Ethoxy und Acetylamino, $X_3$ $C_1$-$C_8$-Alkyl oder Phenyl bedeutet, wenn n 1 ist, $R_3$ eine Gruppe der Formeln IIIb bis IIIg bedeutet,

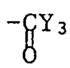 , 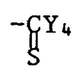 , 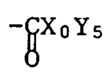 , 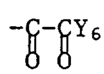 , 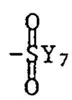 , 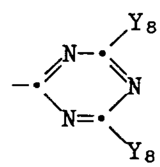 ,

(IIIb) (IIIc) (IIId) (IIIe) (IIIf) (IIIg)

$X_0$ ein Sauerstoffatom oder Schwefelatom ist, $Y_3$ $C_4$-$C_{20}$-Alkyl, $C_3$-$C_{20}$-Alkenyl, Phenyl, durch $C_1$-$C_4$-Alkyl, Chlor, $-NO_2$, Methoxy oder/und Ethoxy substituiertes Phenyl, 2-Phenylethenyl, Di($C_1$-$C_4$-alkyl)-amino, Diphenylamino, $C_1$-$C_{20}$-Alkylamino, $C_3$-$C_8$-Cycloalkylamino, Phenylamino, am Phenylring durch $C_1$-$C_4$-Alkyl, Chlor, Hydroxy, Methoxy oder/und Ethoxy substituiertes Phenylamino, Benzylamino, Benzolsulfonamido oder Toluolsulfonamido darstellt, $Y_4$ Di($C_1$-$C_4$-alkyl)amino, Diphenylamino, $C_1$-$C_8$-Alkylamino, Phenylamino, am Phenylring durch $C_1$-$C_4$-Alkyl, Chlor, Hydroxy, Methoxy oder/und Ethoxy substituiertes Phenylamino oder Benzylamino ist, $Y_5$ $C_1$-$C_{20}$-Alkyl, durch ein oder zwei Sauerstoffatome unterbrochenes $C_3$-$C_6$-Alkyl, $C_5$-$C_{12}$-Cycloalkyl, durch $C_1$-$C_4$-Alkyl substituiertes $C_5$-$C_{12}$-Cycloalkyl, Phenyl oder am Phenylrest durch $C_1$-$C_{20}$-Alkyl substituiertes $C_7$-$C_{10}$-Phenylalkyl bedeutet, $Y_6$ $C_1$-$C_4$-Alkoxy, Phenylamino oder an der Phenylgruppe durch $C_1$-$C_4$-Alkyl, Chlor, Hydroxy, Methoxy oder/und Ethoxy substituiertes Phenylamino ist, $Y_7$ $C_1$-$C_4$-Alkyl, Phenyl oder durch $C_1$-$C_4$-Alkyl, Chlor, $-NO_2$, ($C_1$-$C_{12}$-Alkyl)oxycarbonyl und/oder Phenyloxycarbonyl substituiertes Phenyl darstellt, die Reste $Y_8$ unabhängig voneinander $C_1$-$C_4$-Alkoxy oder Allyloxy bedeuten, und wenn n 2 ist, $R_3$ eine Gruppe der Formel IVa, IVb, IVc oder IVd darstellt,

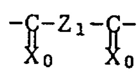 , 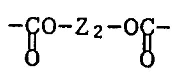 , 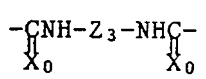 , 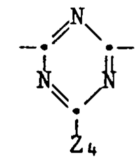 ,

(IVa) (IVb) (IVc) (IVd)

$X_0$ die oben angegebene Bedeutung besitzt,
$Z_1$ eine direkte Bindung, $C_1$-$C_{12}$-Alkylen oder Phenylen bedeutet, $Z_2$ $C_2$-$C_{12}$-Alkylen oder 3-Oxapentylen ist, $Z_3$ $C_4$-$C_8$-Alkylen oder Phenylen und $Z_4$ $C_1$-$C_4$-Alkoxy oder Allyloxy darstellen, mit der Maßgabe, daß $R_3$ nicht für Benzoyl, $C_4$-Alkanoyl, $C_1$-$C_4$-Alkylsulfonyl, Phenylsulfonyl oder Tolylsulfonyl steht, wenn $R_2$ $C_2$-$C_4$-Alkanoyl oder Benzoyl bedeutet.

**16.** Verbindungen gemäss Anspruch 15, worin n 1 oder 2 ist, $R_1$ $C_1$-$C_4$-Alkyl bedeutet, $R_2$ eine Gruppe der Formel IIa, IIb, IIc oder IId darstellt, $X_1$ $C_1$-$C_{20}$-Alkyl, durch ein oder zwei Sauerstoffatome oder Schwefelatome unterbrochenes oder/und durch OH substituiertes $C_3$-$C_6$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_{10}$-Alkyl, Chlor, Methoxy oder/und Ethoxy substituiertes Phenyl, $C_7$-$C_{10}$-Phenylalkyl oder am Phenylrest durch $C_1$-$C_{20}$-Alkyl substituiertes $C_7$-$C_{10}$-Phenylalkyl ist, $X_2$ Phenyl oder durch 1 bis 3 Reste substituiertes Phenyl darstellt, wobei die Reste ausgewählt werden aus der Gruppe bestehend aus $C_1$-$C_4$-Alkyl, Chlor, Hydroxy, Methoxy, Ethoxy und Acetylamino, $X_3$ $C_1$-$C_8$-Alkyl oder Phenyl bedeutet, wenn n 1 ist, $R_3$ eine Gruppe der Formeln IIIb bis IIIg bedeutet, $X_0$ ein Sauerstoffatom ist, $Y_3$ $C_4$-$C_{20}$-Alkyl, $C_3$-$C_{20}$-Alkenyl, Phenyl, durch $C_1$-$C_4$-Alkyl, Chlor, Methoxy oder/und Ethoxy substituiertes Phenyl, 2-Phenylethenyl, Di($C_1$-$C_4$-alkyl)amino, $C_1$-$C_8$-Alkylamino, Phenylamino, Benzylamino, Benzolsulfonamido oder Toluolsulfonamido darstellt, $Y_4$ Di($C_1$-$C_4$-alkyl)amino, $C_1$-$C_8$-Alkylamino, Phenylamino oder Benzylamino ist, $Y_5$ $C_1$-$C_{20}$-Alkyl, durch ein oder zwei Sauerstoffatome unterbrochenes $C_3$-$C_6$-Alkyl, $C_5$-$C_{12}$-Cycloalkyl, durch $C_1$-$C_4$-Alkyl substituiertes $C_5$-$C_{12}$-Cycloalkyl, Phenyl oder am Phenylrest durch $C_1$-$C_{20}$-Alkyl substituiertes $C_7$-$C_{10}$-Phenylalkyl bedeutet, $Y_6$ $C_1$-$C_4$-Alkoxy, Phenylamino oder an der Phenylgruppe durch $C_1$-$C_4$-Alkyl, Chlor, Methoxy oder/und Ethoxy substituiertes Phenylamino ist, $Y_7$ Phenyl oder Tolyl darstellt, die Reste $Y_8$ unabhängig voneinander $C_1$-$C_4$-Alkoxy oder Allyloxy bedeuten, und wenn n 2 ist, $R_3$ eine Gruppe der Formel IVa, IVb, IVc oder IVd darstellt, $X_0$ die oben angegebene Bedeutung hat, $Z_1$ eine direkte Bindung, $C_1$-$C_{12}$-Alkylen oder Phenylen bedeutet, $Z_2$ $C_2$-$C_{12}$-Alkylen oder 3-Oxapentylen ist, $Z_3$ $C_4$-$C_8$-Alkylen oder Phenylen und $Z_4$ $C_1$-$C_4$-Alkoxy oder Allyloxy darstellen.

**17.** Verbindungen gemäss Anspruch 15, worin $X_1$ $C_1$-$C_{18}$-Alkyl, durch ein oder zwei Sauerstoffatome oder Schwefelatome unterbrochenes oder/und durch OH substituiertes $C_3$-$C_6$-Alkyl, Allyl, Methallyl, Oleyl, $C_5$-$C_8$-Cycloalkyl, Phenyl, durch $C_1$-$C_{10}$-Alkyl, Chlor, Methoxy oder/und Ethoxy substituiertes Phenyl, $C_7$-$C_{10}$-Phenylalkyl oder am Phenylrest durch $C_8$-$C_{14}$-Alkyl substituiertes $C_7$-$C_{10}$-Phenylalkyl ist, $X_3$ Methyl, Ethyl oder Phenyl bedeutet, $Y_3$ $C_4$-$C_{18}$-Alkyl, Allyl, Methallyl, Oleyl, Phenyl, durch $C_1$-$C_4$-Alkyl, Chlor, Methoxy oder/und Ethoxy substituiertes Phenyl, 2-Phenylethenyl oder Di($C_1$-$C_4$-alkyl)amino ist und $Y_5$ $C_1$-$C_{18}$-Alkyl, $C_5$-$C_8$-Cycloalkyl, Phenyl oder am Phenylrest durch $C_8$-$C_{14}$-Alkyl substituiertes $C_7$-$C_{10}$-Phenylalkyl darstellt.

**18.** Die Verbindung 2-Methyl-3-methoxycarbonyl-4-ethoxycarbonylaminopyrrol, 2-Methyl-3-methoxycarbonyl-4-benzoylaminopyrrol, 2-Methyl-3-methoxycarbonyl-4-(2',4'-diallyloxy-1',3',5'-triazin-6'-yl)-aminopyrrol 1,4-Bis[(2'-methyl-3'-methoxycarbonylpyrrol-4'-yl)carbamoyl]butan oder 1,6-Bis[(2'-methyl-3'-methoxycarbonylpyrrol-4'-yl)carbamoyloxy]hexan gemäss Anspruch 15.

## Claims

1. A composition comprising a) a chlorine-containing polymer and b) at least one compound of formula I

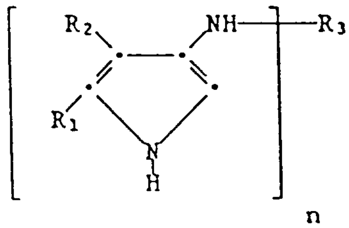

(I)

wherein n is 1 or 2, $R_1$ is $C_1$-$C_4$alkyl, $R_2$ is a group of formula IIa, IIb, IIc or IId

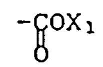 , 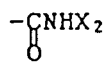 , 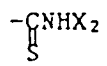 , 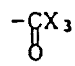 ,

(IIa) (IIb) (IIc) (IId)

$X_1$ is hydrogen, $C_1$-$C_{20}$alkyl, $C_3$-$C_6$alkyl interrupted by one or two oxygen atoms or sulfur atoms or/and substituted by OH, $C_3$-$C_{20}$alkenyl, $C_5$-$C_{12}$cycloalkyl, $C_5$-$C_{12}$cycloalkyl substituted by $C_1$-$C_4$alkyl, phenyl, phenyl substituted by $C_1$-$C_{10}$alkyl, chlorine, hydroxy, methoxy or/and by ethoxy, $C_7$-$C_{10}$phenylalkyl or $C_7$-$C_{10}$phenylalkyl substituted in the phenyl radical by $C_1$-$C_{20}$alkyl, chlorine, hydroxy, methoxy or/and by ethoxy, $X_2$ is phenyl or phenyl substituted by from 1 to 3 radicals, the radicals being selected from the group consisting of $C_1$-$C_4$alkyl, chlorine, hydroxy, methoxy, ethoxy and acetylamino, $X_3$ is $C_1$-$C_8$alkyl or phenyl, when n is 1 $R_3$ is a group of formula IIIa to IIIg

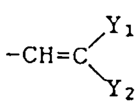 , 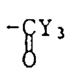 , 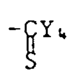 , 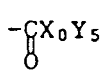 , 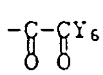 , 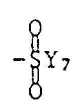 ,

(IIIa) (IIIb) (IIIc) (IIId) (IIIe) (IIIf)

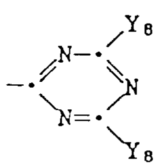 ,

(IIIg)

$X_0$ is an oxygen atom or a sulfur atom, $Y_1$ and $Y_2$ are each independently of the other -CN, benzoyl, $C_2$-$C_4$alkanoyl or $C_2$-$C_4$alkoxycarbonyl, $Y_3$ is $C_1$-$C_{20}$alkyl, $C_3$-$C_{20}$alkenyl,phenyl, phenyl substituted by $C_1$-$C_4$alkyl, chlorine, $-NO_2$, methoxy or/and by ethoxy, 2-phenylethenyl, di($C_1$-$C_4$alkyl)amino, diphenylamino, $C_1$-$C_{20}$alkylamino, $C_3$-$C_8$cycloalkylamino, phenylamino, phenylamino substituted in the phenyl ring by $C_1$-$C_4$alkyl, chlorine, hydroxy, methoxy or/and by ethoxy, benzylamino, benzenesulfonamido or toluenesulfonamido, $Y_4$ is di($C_1$-$C_4$alkyl)amino, diphenylamino, $C_1$-$C_8$alkylamino, phenylamino, phenylamino substituted in the phenyl ring by $C_1$-$C_4$alkyl, chlorine, hydroxy, methoxy or/and by ethoxy, or benzylamino, $Y_5$ is $C_1$-$C_{20}$alkyl, $C_3$-$C_6$alkyl interrupted by one or two oxygen atoms, $C_5$-$C_{12}$-cycloalkyl, $C_5$-$C_{12}$cycloalkyl substituted by $C_1$-$C_4$alkyl, phenyl, $C_7$-$C_{10}$phenylalkyl or $C_7$-$C_{10}$phenylalkyl substituted in the phenyl radical by $C_1$-$C_{20}$alkyl, $Y_6$ is $C_1$-$C_4$alkoxy, phenylamino or phenylamino substituted in the phenyl group by $C_1$-$C_4$alkyl, chlorine, hydroxy, methoxy or/and by ethoxy, $Y_7$ is $C_1$-$C_4$alkyl, phenyl or phenyl substituted by $C_1$-$C_4$-alkyl, chlorine, $-NO_2$, ($C_1$-$C_{12}$alkyl)-oxycarbonyl and/or by phenoxycarbonyl, the radicals $Y_8$ are each independently of the other $C_1$-$C_4$alkoxy or allyloxy, and when n is 2 $R_3$ is a group of formula IVa, IVb, IVc or IVd

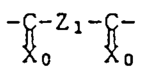 , 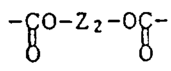 , 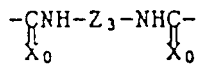 , 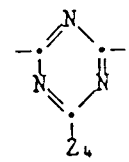 ,

(IVa) (IVb) (IVc) (IVd)

$X_0$ is as defined above, $Z_1$ is a direct bond, $C_1$-$C_{12}$alkylene or phenylene, $Z_2$ is $C_2$-$C_{12}$alkylene or 3-oxapentylene, $Z_3$ is $C_4$-$C_8$alkylene or phenylene and $Z_4$ is $C_1$-$C_4$alkoxy or allyloxy.

**2.** A composition according to claim 1, wherein n is 1 or 2, $R_1$ is $C_1$-$C_4$alkyl, $R_2$ is a group of formula IIa, IIb, IIc or IId, $X_1$ is $C_1$-$C_{20}$alkyl, $C_3$-$C_6$alkyl interrupted by one or two oxygen atoms or sulfur atoms or/and substituted by OH, $C_3$-$C_{20}$alkenyl, $C_5$-$C_{12}$cycloalkyl, $C_5$-$C_{12}$cycloalkyl substituted by $C_1$-$C_4$alkyl, phenyl, phenyl substituted by $C_1$-$C_{10}$alkyl, chlorine, methoxy or/and by ethoxy, $C_7$-$C_{10}$-phenylalkyl or $C_7$-$C_{10}$phenylalkyl substituted in the phenyl radical by $C_1$-$C_{20}$alkyl, $X_2$ is phenyl or phenyl substituted by from 1 to 3 radicals, the radicals being selected from the group consisting of $C_1$-$C_4$alkyl, chlorine, hydroxy, methoxy, ethoxy and acetylamino, $X_3$ is $C_1$-$C_8$alkyl or phenyl, when n is 1 $R_3$ is a group of formula IIIa to IIIg, $X_0$ is an oxygen atom, $Y_1$ and $Y_2$ are each independently of the other -CN, benzoyl, $C_2$-$C_4$alkanoyl or $C_2$-$C_4$alkoxycarbonyl, $Y_3$ is $C_1$-$C_{20}$alkyl, $C_3$-$C_{20}$alkenyl, phenyl, phenyl substituted by $C_1$-$C_4$alkyl, chlorine, methoxy or/and by ethoxy, 2-phenylethenyl, di($C_1$-$C_4$alkyl)amino, $C_1$-$C_8$alkylamino, phenylamino, benzylamino, benzenesulfonamido or toluenesulfonamido, $Y_4$ is di($C_1$-$C_4$alkyl)amino, $C_1$-$C_8$alkylamino, phenylamino or benzylamino, $Y_5$ is $C_1$-$C_{20}$alkyl, $C_3$-$C_6$alkyl interrupted by one or two oxygen atoms, $C_5$-$C_{12}$-cycloalkyl, $C_5$-$C_{12}$cycloalkyl substituted by $C_1$-$C_4$alkyl, phenyl, $C_7$-$C_{10}$phenylalkyl or $C_7$-$C_{10}$phenylalkyl substituted in the phenyl radical by $C_1$-$C_{20}$alkyl, $Y_6$ is $C_1$-$C_4$alkoxy, phenylamino or phenylamino substituted in the phenyl group by $C_1$-$C_4$alkyl, chlorine, methoxy or/and by ethoxy, $Y_7$ is phenyl or tolyl, the radicals $Y_8$ are each independently of the other $C_1$-$C_4$alkoxy or allyloxy, and when n is 2 $R_3$ is a group of formula IVa, IVb, IVc or IVd, $X_0$ is as defined above, $Z_1$ is a direct bond, $C_1$-$C_{12}$alkylene or phenylene, $Z_2$ is $C_2$-$C_{12}$alkylene or 3-oxapentylene, $Z_3$ is $C_4$-$C_8$alkylene or phenylene and $Z_4$ is $C_1$-$C_4$alkoxy or allyloxy.

**3.** A composition according to claim 1, wherein $X_1$ is $C_1$-$C_{18}$alkyl, $C_3$-$C_6$alkyl interrupted by one or two oxygen atoms or sulfur atoms or/and substituted by OH, allyl, methallyl, oleyl, $C_5$-$C_8$cycloalkyl, phenyl, phenyl substituted by $C_1$-$C_{10}$alkyl, chlorine, methoxy or/and by ethoxy, $C_7$-$C_{10}$phenylalkyl or $C_7$-$C_{10}$phenylalkyl substituted in the phenyl radical by $C_8$-$C_{14}$alkyl, $X_3$ is methyl, ethyl or phenyl, $Y_3$ is $C_1$-$C_{18}$alkyl, allyl, methallyl, oleyl, phenyl, phenyl substituted by $C_1$-$C_4$alkyl, chlorine, methoxy or/and by ethoxy, 2-phenylethenyl or di($C_1$-$C_4$alkyl)amino, and $Y_5$ is $C_1$-$C_{18}$alkyl, $C_5$-$C_8$cycloalkyl, phenyl, $C_7$-$C_{10}$-phenylalkyl or $C_7$-$C_{10}$phenylalkyl substituted in the phenyl radical by $C_8$-$C_{14}$alkyl.

**4.** A composition according to claim 1, wherein $R_1$ is methyl.

**5.** A composition according to claim 1, wherein n is 1, $R_2$ is a group of formula IIa or IIc, and $R_3$ is a group of formula IIIb, IIId or IIIg.

**6.** A composition according to claim 1, wherein n is 1, $R_1$ is methyl, $R_2$ is a group of formula IIa, $X_1$ is $C_1$-$C_{12}$alkyl, $R_3$ is a group of formula IIIb, IIId or IIIg, $Y_3$ is $C_1$-$C_{18}$-alkyl, allyl, phenyl or phenyl substituted by $C_1$-$C_4$alkyl, chlorine, methoxy or/and by ethoxy, and $Y_5$ is $C_1$-$C_{18}$alkyl, cyclohexyl, phenyl or benzyl.

**7.** A composition according to claim 1, wherein n is 2.

**8.** A composition according to claim 1, wherein n is 2 and $R_3$ is a group of formula IVa or IVb.

**9.** A composition according to claim 1, wherein $R_2$ is a group of formula IIa.

**10.** A composition according to claim 1, wherein the compound of formula I is 2-methyl-3-methoxycarbonyl-4-ethoxycarbonylaminopyrrole, 2-methyl-3-methoxycarbonyl-4-benzoylaminopyrrole, 2-methyl-3-methoxycarbonyl-4-(2',4'-diallyloxy-1',3',5'-triazin-6'-yl)aminopyrrole, 1,4-bis[(2'-methyl-3'-methoxycarbonylpyrrol-4'-yl)carbamoyl]butane or 1,6-bis[(2'-methyl-3'-methoxycarbonylpyrrol-4'-yl)carbamoyloxy]-hexane.

**11.** A composition according to claim 1, wherein the chlorine-containing polymer is polyvinyl chloride.

**12.** A composition according to claim 1 comprising, in addition, at least one Me(II) carboxylate and/or Me-(II) phenolate wherein Me(II) is Ba, Ca, Mg, Cd or Zn.

**13.** A composition according to claim 1 comprising, in addition, an epoxy compound and/or a phosphite.

**14.** The use of a compound of formula I according to claim 1 for stabilising a chlorine-containing polymer against thermal and light-induced degradation.

**15.** A compound of formula Ia

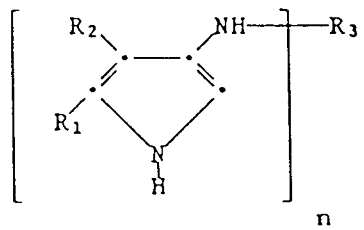

(Ia)

wherein n is 1 or 2, $R_1$ is $C_1$-$C_4$alkyl, $R_2$ is a group of formula IIa, IIb, IIc or IId

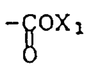 , 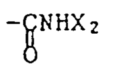 , 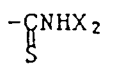 , 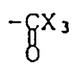 ,

(IIa) (IIb) (IIc) (IId)

$X_1$ is hydrogen, $C_1$-$C_{20}$alkyl, $C_3$-$C_6$alkyl interrupted by one or two oxygen atoms or sulfur atoms or/and substituted by OH, $C_3$-$C_{20}$alkenyl, $C_5$-$C_{12}$cycloalkyl, $C_5$-$C_{12}$cycloalkyl substituted by $C_1$-$C_4$alkyl, phenyl, phenyl substituted by $C_1$-$C_{10}$alkyl, chlorine, hydroxy, methoxy or/and by ethoxy, $C_7$-$C_{10}$phenylalkyl or $C_7$-$C_{10}$phenylalkyl substituted in the phenyl radical by $C_1$-$C_{20}$alkyl, chlorine, hydroxy, methoxy or/and by ethoxy, $X_2$ is phenyl or phenyl substituted by from 1 to 3 radicals, the radicals being selected from the group consisting of $C_1$-$C_4$alkyl, chlorine, hydroxy, methoxy, ethoxy and acetylamino, $X_3$ is $C_1$-$C_8$alkyl or phenyl, when n is 1 $R_3$ is a group of formula IIIb to IIIg

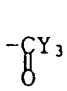 , 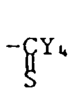 , 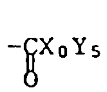 , 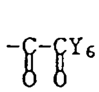 , 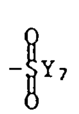 , 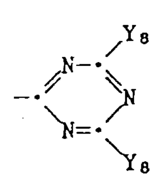 ,

(IIIb) (IIIc) (IIId) (IIIe) (IIIf) (IIIg)

$X_0$ is an oxygen atom or a sulfur atom, $Y_3$ is $C_4$-$C_{20}$alkyl, $C_3$-$C_{20}$alkenyl, phenyl, phenyl substituted by $C_1$-$C_4$alkyl, chlorine, $-NO_2$, methoxy or/and by ethoxy, 2-phenylethenyl, di($C_1$-$C_4$alkyl)amino, diphenylamino, $C_1$-$C_{20}$alkylamino, $C_3$-$C_8$cycloalkylamino, phenylamino, phenylamino substituted in the phenyl ring by $C_1$-$C_4$alkyl, chlorine, hydroxy, methoxy or/and by ethoxy, benzylamino, benzenesulfonamido or toluenesulfonamido, $Y_4$ is di($C_1$-$C_4$alkyl)amino, diphenylamino, $C_1$-$C_8$alkylamino, phenylamino, phenylamino substituted in the phenyl ring by $C_1$-$C_4$alkyl, chlorine, hydroxy, methoxy or/and by ethoxy, or benzylamino, $Y_5$ is $C_1$-$C_{20}$-alkyl, $C_3$-$C_6$alkyl interrupted by one or two oxygen atoms, $C_5$-$C_{12}$cycloalkyl, $C_5$-$C_{12}$cycloalkyl substituted by $C_1$-$C_4$-alkyl, phenyl, or $C_7$-$C_{10}$phenylalkyl

substituted in the phenyl radical by $C_1$-$C_{20}$alkyl, $Y_6$ is $C_1$-$C_4$alkoxy, phenylamino or phenylamino substituted in the phenyl group by $C_1$-$C_4$alkyl, chlorine, hydroxy, methoxy or/and by ethoxy, $Y_7$ is $C_1$-$C_4$alkyl, phenyl or phenyl substituted by $C_1$-$C_4$alkyl, chlorine, -$NO_2$, ($C_1$-$C_{12}$alkyl)oxycarbonyl and/or by phenoxycarbonyl, the radicals $Y_8$ are each independently of the other $C_1$-$C_4$alkoxy or allyloxy, and when n is 2 $R_3$ is a group of formula IVa, IVb, IVc or IVd

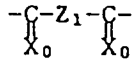 , 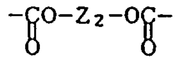 , 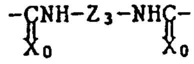 , 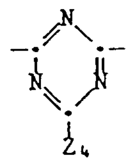 ,

(IVa) (IVb) (IVc) (IVd)

$X_0$ is as defined above, $Z_1$ is a direct bond, $C_1$-$C_{12}$alkylene or phenylene, $Z_2$ is $C_2$-$C_{12}$alkylene or 3-oxapentylene, $Z_3$ is $C_4$-$C_8$alkylene or phenylene and $Z_4$ is $C_1$-$C_4$alkoxy or allyloxy, with the proviso that $R_3$ is not benzoyl, $C_4$alkenoyl, $C_1$-$C_4$alkylsulfonyl, phenylsulfonyl or tolylsulfonyl when $R_2$ is $C_2$-$C_4$alkanoyl or benzoyl.

**16.** A compound according to claim 15, wherein n is 1 or 2, $R_1$ is $C_1$-$C_4$alkyl, $R_2$ is a group of formula IIa, IIb, IIc or IId, $X_1$ is $C_1$-$C_{20}$alkyl, $C_3$-$C_6$alkyl interrupted by one or two oxygen atoms or sulfur atoms or/and substituted by OH, $C_3$-$C_{20}$alkenyl, $C_5$-$C_{12}$cycloalkyl, $C_5$-$C_{12}$cycloalkyl substituted by $C_1$-$C_4$alkyl, phenyl, phenyl substituted by $C_1$-$C_{10}$alkyl, chlorine, methoxy or/and by ethoxy, $C_7$-$C_{10}$-phenylalkyl or $C_7$-$C_{10}$phenylalkyl substituted in the phenyl radical by $C_1$-$C_{20}$alkyl, $X_2$ is phenyl or phenyl substituted by from 1 to 3 radicals, the radicals being selected from the group consisting of $C_1$-$C_4$alkyl, chlorine, hydroxy, methoxy, ethoxy and acetylamino, $X_3$ is $C_1$-$C_8$alkyl or phenyl, when n is 1 $R_3$ is a group of formula IIIb to IIIg, $X_0$ is an oxygen atom, $Y_3$ is $C_4$-$C_{20}$alkyl, $C_3$-$C_{20}$alkenyl, phenyl, phenyl substituted by $C_1$-$C_4$alkyl, chlorine, methoxy or/and by ethoxy, 2-phenylethenyl, di($C_1$-$C_4$alkyl)amino, $C_1$-$C_8$alkylamino, phenylamino, benzylamino, benzenesulfonamido or toluenesulfonamido, $Y_4$ is di($C_1$-$C_4$alkyl)amino, $C_1$-$C_8$alkylamino, phenylamino or benzylamino, $Y_5$ is $C_1$-$C_{20}$-alkyl, $C_3$-$C_6$alkyl interrupted by one or two oxygen atoms, $C_5$-$C_{12}$cycloalkyl, $C_5$-$C_{12}$cycloalkyl substituted by $C_1$-$C_4$-alkyl, phenyl, or $C_7$-$C_{10}$phenylalkyl substituted in the phenyl radical by $C_1$-$C_{20}$alkyl, $Y_6$ is $C_1$-$C_4$alkoxy, phenylamino or phenylamino substituted in the phenyl group by $C_1$-$C_4$alkyl, chlorine, methoxy or/and by ethoxy, $Y_7$ is phenyl or tolyl, the radicals $Y_8$ are each independently of the other $C_1$-$C_4$alkoxy or allyloxy, and when n is 2 $R_3$ is a group of formula IVa, IVb, IVc or IVd, $X_0$ is as defined above, $Z_1$ is a direct bond, $C_1$-$C_{12}$alkylene or phenylene, $Z_2$ is $C_2$-$C_{12}$alkylene or 3-oxapentylene, $Z_3$ is $C_4$-$C_8$alkylene or phenylene and $Z_4$ is $C_1$-$C_4$alkoxy or allyloxy.

**17.** A compound according to claim 15, wherein $X_1$ is $C_1$-$C_{18}$alkyl, $C_3$-$C_6$alkyl interrupted by one or two oxygen atoms or sulfur atoms or/and substituted by OH, allyl, methallyl, oleyl, $C_5$-$C_8$cycloalkyl, phenyl, phenyl substituted by $C_1$-$C_{10}$alkyl, chlorine, methoxy or/and by ethoxy, $C_7$-$C_{10}$phenylalkyl or $C_7$-$C_{10}$phenylalkyl substituted in the phenyl radical by $C_8$-$C_{14}$alkyl, $X_3$ is methyl, ethyl or phenyl, $Y_3$ is $C_4$-$C_{18}$alkyl, allyl, methallyl, oleyl, phenyl, phenyl substituted by $C_1$-$C_4$alkyl, chlorine, methoxy or/and by ethoxy, 2-phenylethenyl or di($C_1$-$C_4$alkyl)amino, and $Y_5$ is $C_1$-$C_{18}$alkyl, $C_5$-$C_8$cycloalkyl, phenyl, or $C_7$-$C_{10}$phenylalkyl substituted in the phenyl radical by $C_8$-$C_{14}$alkyl.

**18.** The compound 2-methyl-3-methoxycarbonyl-4-ethoxycarbonylaminopyrrole, 2-methyl-3-methoxycarbonyl-4-benzoylaminopyrrole, 2-methyl-3-methoxycarbonyl-4-(2',4'-diallyloxy-1',3',5'-triazin-6'-yl)-aminopyrrole, 1,4-bis[(2'-methyl-3'-methoxycarbonylpyrrol-4'-yl)carbamoyl]butane or 1,6-bis[(2'-methyl-3'-methoxycarbonylpyrrol-4'-yl)carbamoyloxy]hexane according to claim 15.

## Revendications

**1.** Compositions contenant a) un polymérisat chloré et b) au moins un composé de formule I,

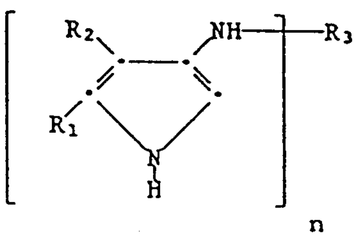

(I)

dans laquelle n vaut 1 ou 2, $R_1$ signifie un alkyle en $C_1$-$C_4$, $R_2$ représente un groupe de formule IIa, IIb, IIc ou IId,

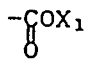 , 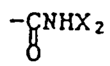 , 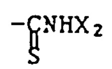 , 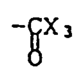 ,

(IIa) (IIb) (IIc) (IId)

$X_1$ est un hydrogène, un alkyle en $C_1$-$C_{20}$, un alkyle en $C_3$-$C_6$ interrompu par un ou deux atomes d'oxygène ou de soufre ou/et substitué par OH, un alcényle en $C_3$-$C_{20}$, un cycloalkyle en $C_5$-$C_{12}$, un cycloalkyle en $C_5$-$C_{12}$ substitué par des alkyles en $C_1$-$C_4$, un phényle, un phényle substitué par des alkyles en $C_1$-$C_{10}$, chlore, hydroxy, méthoxy ou/et éthoxy, un phénylalkyle en $C_7$-$C_{10}$ ou un phénylalkyle en $C_7$-$C_{10}$ substitué sur le reste phényle par des alkyles en $C_1$-$C_{20}$, chlore, hydroxy, méthoxy ou/et éthoxy, $X_2$ représente un phényle ou un phényle substitué par 1 à 3 restes, les restes étant choisis parmi le groupe formé des alkyles en $C_1$-$C_4$,chlore, hydroxy, méthoxy, éthoxy et acétylamino, $X_3$ signifie un alkyle en $C_1$-$C_8$ ou un phényle, lorsque n vaut 1, $R_3$ signifie un groupe des formules IIIa à IIIg,

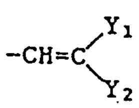 , 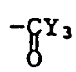 , 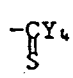 , 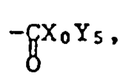 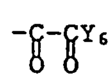 , 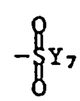 ,

(IIIa) (IIIb) (IIIc) (IIId) (IIIe) (IIIf)

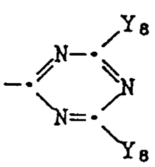 ,

(IIIg)

$X_0$ est un atome d'oxygène ou de soufre, $Y_1$ et $Y_2$ sont, indépendamment l'un de l'autre, des -CN, des benzoyles, des alcanoyles en $C_2$-$C_4$ ou des alcoxycarbonyles en $C_2$-$C_4$, $Y_3$ représente un alkyle en $C_1$-$C_{20}$, un alcényle en $C_3$-$C_{20}$, un phényle, un phényle substitué par des alkyles en $C_1$-$C_4$,chlore, $-NO_2$, méthoxy ou/et éthoxy, un 2-phényléthènyle, un di-(alkyl en $C_1$-$C_4$)-amino, un diphénylamino, un (alkyl en $C_1$-$C_{20}$)-amino, un (cycloalkyl en $C_3$-$C_8$)-amino, un phénylamino, un benzène-sulfonamido, un toluène-sulfonamido, ou un phénylamino substitué sur le cycle phényle par des alkyles en $C_1$-$C_4$, chlore, hydroxy, méthoxy ou/et éthoxy, un benzylamino, $Y_4$ est un di-(alkyl en $C_1$-$C_4$)-amino, un diphénylamino, un (alkyle en $C_1$-$C_8$)-amino, un phénylamino, un benzylamino ou un phénylamino substitué sur le cycle phényle par des alkyles en $C_1$-$C_4$, chlore, hydroxy, méthoxy ou/et éthoxy, $Y_5$ signifie un alkyle en $C_1$-$C_{20}$, un alkyle en $C_3$-$C_6$ interrompu par un ou deux atomes d'oxygène, un

cycloalkyle en $C_5$-$C_{12}$, un cycloalkyle en $C_5$-$C_{12}$ substitué par des alkyles en $C_1$-$C_4$, un phényle, un phénylalkyle en $C_7$-$C_{10}$ ou un phénylalkyle en $C_7$-$C_{10}$ substitué sur le reste phényle par des alkyles en $C_1$-$C_{20}$, $Y_6$ est un alcoxy en $C_1$-$C_4$, un phénylamino ou un phénylamino substitué sur le groupe phényle par des alkyles en $C_1$-$C_4$, chlore, hydroxy, méthoxy ou/et éthoxy, $Y_7$ est un alkyle en $C_1$-$C_4$, un phényle ou un phényle substitué par des alkyles en $C_1$-$C_4$, chlore, $-NO_2$, (alkyl en $C_1$-$C_{12}$)-oxycarbonyle et/ou phényloxycarbonyle, les restes $Y_8$ signifient, indépendamment l'un de l'autre, des alcoxy en $C_1$-$C_4$ ou des allyloxy, et lorsque n vaut 2, $R_3$ représente un groupe de formule IVa, IVb, IVc ou IVd,

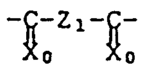
(IVa)
,
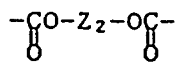
(IVb)
,
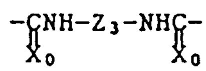
(IVc)
,
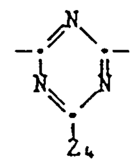
(IVd)
,

$X_0$ possède la signification donnée ci-dessus, $Z_1$ signifie une liaison directe, un alkylène en $C_1$-$C_{12}$ ou un phénylène, $Z_2$ est un alkylène en $C_2$-$C_{12}$ ou un 3-oxapentylène, $Z_3$ représente un alkylène en $C_4$-$C_8$ ou un phénylène et $Z_4$ représente un alcoxy en $C_1$-$C_4$ ou un allyloxy.

**2.** Compositions selon la revendication 1, dans lesquelles n vaut 1 ou 2, $R_1$ signifie un alkyle en $C_1$-$C_4$, $R_2$ représente un groupe de formule IIa, IIb, IIc ou IId, $X_1$ est un alkyle en $C_1$-$C_{20}$, un alkyle en $C_3$-$C_6$ interrompu par un ou deux atomes d'oxygène ou de soufre ou/et substitué par OH, un alcényle en $C_3$-$C_{20}$, un cycloalkyle en $C_5$-$C_{12}$, un cycloalkyle en $C_5$-$C_{12}$ substitué par des alkyles en $C_1$-$C_4$, un phényle, un phényle substitué par des alkyles en $C_1$-$C_{10}$,chlore, méthoxy ou/et éthoxy, un phénylalkyle en $C_7$-$C_{10}$ ou un phénylalkyle en $C_7$-$C_{10}$ substitué sur le reste phényle par des alkyles en $C_1$-$C_{20}$, $X_2$ représente un phényle ou un phényle substitué par 1 à 3 restes, les restes étant choisis parmi le groupe formé des alkyles en $C_1$-$C_4$, chlore, hydroxy, méthoxy, éthoxy et acétylamino, $X_3$ signifie un alkyle en $C_1$-$C_8$ ou un phényle, lorsque n vaut 1, $R_3$ signifie un groupe de formules IIIa à IIIg, $X_0$ est un atome d'oxygène, $Y_1$ et $Y_2$ sont, indépendamment l'un de l'autre, des -CN, des benzoyles, des alcanoyles en $C_2$-$C_4$ ou des alcoxycarbonyles en $C_2$-$C_4$, $Y_3$ représente un alkyle en $C_1$-$C_{20}$, un alcényle en $C_3$-$C_{20}$, un phényle, un benzène-sulfonamido, un toluène-sulfonamido ou un phényle substitué par des alkyles en $C_1$-$C_4$, chlore, méthoxy ou/et éthoxy, un 2-phényléthényle, un di-(alkyl en $C_1$-$C_4$)-amino, un (alkyl en $C_1$-$C_8$)-amino, un phénylamino, un benzylamino, $Y_4$ est un di(alkyl en $C_1$-$C_4$)-amino, un (alkyl en $C_1$-$C_8$)-amino, un phénylamino, un benzylamino, $Y_5$ est un alkyle en $C_1$-$C_{20}$, un alkyle en $C_3$-$C_6$ interrompu par un ou deux atomes d'oxygène, un cycloalkyle en $C_5$-$C_{12}$, un cycloalkyle en $C_5$-$C_{12}$ substitué par des alkyles en $C_1$-$C_4$, un phényle, un phényalkyle en $C_7$-$C_{10}$ ou un phénylalkyle en $C_7$-$C_{10}$ substitué sur le reste phényle par des alkyles en $C_1$-$C_{20}$, $Y_6$ est un alcoxy en $C_1$-$C_4$, un phénylamino ou un phénylamino substitué sur le groupe phényle par des alkyles en $C_1$-$C_4$, chlore, méthoxy ou/et éthoxy, $Y_7$ représente un phényle ou un tolyle, les restees $Y_8$ signifient, indépendamment l'un de l'autre, des alcoxy en $C_1$-$C_4$ ou des allyloxy, et lorsque n vaut 2, $R_3$ représente un groupe de formules IVa, IVb, IVc ou IVd, $X_O$ a la signification donnée ci-dessus, $Z_1$ signifie une liaison directe, un alkylène en $C_1$-$C_{12}$ ou un phénylène, $Z_2$ est un alkylène en $C_2$-$C_{12}$ ou un 3-oxapentylène, $Z_3$ représente un alkylène en $C_4$-$C_8$ ou un phénylène et $Z_4$ représente un alcoxy en $C_1$-$C_4$ ou un allyloxy.

**3.** Compositions selon la revendication 1, dans lesquelles $X_1$ est un alkyle en $C_1$-$C_{18}$, un alkyle en $C_3$-$C_6$ interrompu par un ou deux atomes d'oxygène ou de soufre ou/et substitué par OH, un allyle, un méthallyle, un oléyle, un cycloalkyle en $C_5$-$C_8$, un phényle, un phényle substitué par des alkyles en $C_1$-$C_{10}$, chlore, méthoxy ou/et éthoxy, un phénylalkyle en $C_7$-$C_{10}$ ou un phénylalkyle en $C_7$-$C_{10}$ substitué sur le reste phényle par des alkyles en $C_8$-$C_{14}$, $X_3$ signifie un méthyle, un éthyle ou un phényle, $Y_3$ est un alkyle en $C_1$-$C_{18}$, un allyle, un méthallyle, un oléyle, un phényle, un phényle substitué par des alkyles en $C_1$-$C_4$,chlore, méthoxy ou/et éthoxy, un 2-phényléthènyle ou un di-(alkyl en $C_1$-$C_4$)-amino et $Y_5$ représente un alkyle en $C_1$-$C_{18}$, un cycloalkyle en $C_5$-$C_8$, un phényle, un phényalkyle en $C_7$-$C_{10}$ ou un phénylalkyle en $C_7$-$C_{10}$ substitué sur le reste phényle par des alkyles en $C_8$-$C_{14}$.

**4.** Compositions selon la revendication 1, dans lesquelles $R_1$ signifie le méthyle.

**5.** Compositions selon la revendication 1, dans lesquelles n signifie 1, $R_2$ est un groupe de formule IIa ou IIc et $R_3$ est un groupe de formule IIIb, IIId ou IIIg.

**6.** Compositions selon la revendication 1, dans lesquelles n signifie 1, $R_1$ est un méthyle, $R_2$ représente un groupe de formule IIa, $X_1$ signifie un alkyle en $C_1$-$C_{12}$, $R_3$ est un groupe de formule IIIb, IIId ou IIIg, $Y_3$ signifie un alkyle en $C_1$-$C_{18}$, un allyle, un phényle ou un phényle substitué par des alkyles en $C_1$-$C_4$, chlore, méthoxy ou/et éthoxy et $Y_5$ représente un alkyle en $C_1$-$C_{18}$, un cyclohexyle, un phényle ou un benzyle.

**7.** Compositions selon la revendication 1, dans lesquelles n signifie 2.

**8.** Compositions selon la revendication 1, dans lesquelles n signifie 2 et $R_3$ est un groupe de formule IVa ou IVb.

**9.** Compositions selon la revendication 1, dans lesquelles $R_2$ est un groupe de formule IIa.

**10.** Compositions selon la revendication 1, dans lesquelles le composé de formule I est : le 2-méthyl-3-méthoxycarbonyl-4-éthoxycarbonylaminopyrrole, le 2-méthyl-3-méthoxycarbonyl-4-benzoylaminopyrrole, le 2-méthyl-3-méthoxycarbonyl-4-(2',4'-diallyloxy-1',3'-5'-triazine-6'-yl)-aminopyrrole, le 1,4-bis-[(2'-methyl-3'-méthoxycarbonylpyrrol-4'-yl)-carbamoyl]-butane ou le 1,6-bis-[(2'-méthyl-3'-méthoxycarbonylpyrrol-4'-yl)-carbamoyl]-hexane

**11.** Compositions selon la revendication 1, dans lesquelles le polymérisat chloré est du chlorure de polyvinyle.

**12.** Compositions selon la revendication 1, contenant, en plus, au moins un carboxylate de Me(II) et/ou un phénolate de Me(II), Me(II) signifiant Ba, Ca, Mg, Cd ou Zn.

**13.** Compositions selon la revendication 1, contenant, en plus, un composé époxy et/ou un phosphite.

**14.** Utilisation des composés de formule I selon la revendication 1 afin de stabiliser un polymérisat chloré contre les dégradations thermiques et les dégradations induites par la lumière.

**15.** Composés de formule Ia,

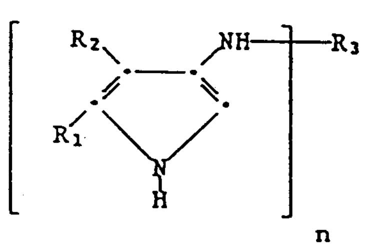

(Ia)

dans laquelle n vaut 1 ou 2, $R_1$ signifie un alkyle en $C_1$-$C_4$, $R^2$ représente un groupe de formule IIa, IIb, IIc ou IId,

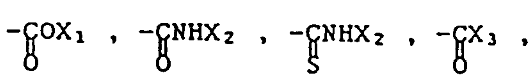

(IIa) (IIb) (IIc) (IId)

$X_1$ est un hydrogène, un alkyle en $C_1$-$C_{20}$, un alkyle en $C_3$-$C_6$ interrompu par un ou deux atomes d'oxygène ou de soufre ou/et substitué par OH, un alcènyle en $C_3$-$C_{20}$, un cycloalkyle en $C_5$-$C_{12}$, un

cycloalkyle en $C_5$-$C_{12}$ substitué par des alkyles en $C_1$-$C_4$, un phényle, un phényle substitué par des alkyles en $C_1$-$C_{10}$, chlore, hydroxy, méthoxy ou/et éthoxy, un phénylalkyle en $C_7$-$C_{10}$ ou un phénylalkyle en $C_7$-$C_{10}$ substitué sur le reste phényle par des alkyles en $C_1$-$C_{20}$, chlore, hydroxy, méthoxy ou/et éthoxy, $X_2$ représente un phényle ou un phényle substitué par 1 à 3 restes, les restes étant choisis parmi le groupe formé des alkyles en $C_1$-$C_4$,chlore, hydroxy, méthoxy, éthoxy et acétylamino, $X_3$ signifie un alkyle en $C_1$-$C_8$ ou un phényle, lorsque n vaut 1, $R_3$ signifie un groupe de formules IIIa à IIIg,

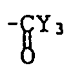 , 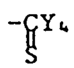 , 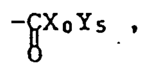 , 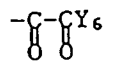 , 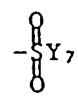 , 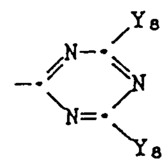 ,

(IIIb) (IIIc) (IIId) (IIIe) (IIIf) (IIIg)

$X_0$ est un atome d'oxygène ou de soufre, $Y_3$ représente un alkyle en $C_4$-$C_{20}$, un alcènyle en $C_3$-$C_{20}$, un phényle, un phényle substitué par des alkyles en $C_1$-$C_4$, chlore, $-NO_2$, méthoxy ou/et éthoxy, un 2-phényléthènyle, un di-(alkyl en $C_1$-$C_4$)-amino, un diphénylamino, un (alkyl en $C_1$-$C_{20}$)-amino, un (cycloalkyl en $C_3$-$C_8$)-amino, un phénylamino, un phénylamino substitué sur le cycle phényle par des alkyles en $C_1$-$C_4$, chlore, hydroxy, méthoxy ou/et éthoxy, un benzylamino, un benzène-sulfonamido ou un toluène-sulfonamido, $Y_4$ est un di(alkyl en $C_1$-$C_4$)-amino, un diphénylamino, un (alkyl en $C_1$-$C_8$)-amino, un phénylamino, un benzylamino ou un phénylamino substitué sur le cycle phényle par des alkyles en $C_1$-$C_4$, chlore, hydroxy, méthoxy ou/et éthoxy, $Y_5$ signifie un alkyle en $C_1$-$C_{20}$, un alkyle en $C_3$-$C_6$ interrompu par un ou deux atomes d'oxygène, un cycloalkyle en $C_5$-$C_{12}$, un cycloalkyle en $C_5$-$C_{12}$ substitué par des alkyles en $C_1$-$C_4$, un phényle ou un phényalkyle en $C_7$-$C_{10}$ substitué sur le reste phényle par des alkyles en $C_1$-$C_{20}$, $Y_6$ est un alcoxy en $C_1$-$C_4$, un phénylamino ou un phénylamino substitué sur le groupe phényle par des alkyles en $C_1$-$C_4$,chlore, hydroxy, méthoxy ou/et éthoxy, $Y_7$ représente un alkyle en $C_1$-$C_4$, un phényle ou un phényle substitué par des alkyles en $C_1$-$C_4$, chlore, $-NO_2$, (alkyl en $C_1$-$C_{12}$)-oxycarbonyle et/ou phényloxycarbonyle, les restes $Y_8$ signifient, indépendamment l'un de l'autre, des alcoxy en $C_1$-$C_4$ ou des allyloxy, et lorsque n vaut 2, $R_3$ représente un groupe de formule IVa, IVb, IVc ou IVd,

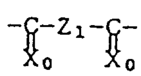 , 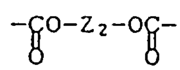 , 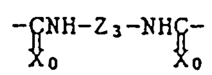 , 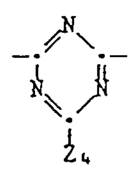 ,

(IVa) (IVb) (IVc) (IVd)

$X_0$ possède la signification donnée ci-dessus, $Z_1$ signifie une liaison directe, un alkylène en $C_1$-$C_{12}$ ou un phénylène, $Z_2$ est un alkylène en $C_2$-$C_{12}$ ou un 3-oxapentylène, $Z_3$ représente un alkylène en $C_4$-$C_8$ ou un phénylène et $Z_4$ représente un alcoxy en $C_1$-$C_4$ ou un allyloxy, à la condition que $R_3$ ne représente pas un benzoyle, un alcénoyle en $C_4$, un alkylsulfonyle en $C_1$-$C_4$, un phénylsulfonyle ou un tolylsulfonyle, lorsque $R_2$ signifie un alcanoyle en $C_2$-$C_4$ ou un benzoyle.

**16.** Composés selon la revendication 15, dans lesquels n vaut 1 ou 2, $R_1$ signifie un alkyle en $C_1$-$C_4$, $R_2$ représente un groupe de formule IIa, IIb, IIc ou IId, $X_1$ est un alkyle en $C_1$-$C_{20}$, un alkyle en $C_3$-$C_6$ interrompu par un ou deux atomes d'oxygène ou de soufre ou/et substitué par OH, un alcènyle en $C_3$-$C_{20}$, un cycloalkyle en $C_5$-$C_{12}$, un cycloalkyle en $C_5$-$C_{12}$ substitué par des alkyles en $C_1$-$C_4$, un phényle, un phényle substitué par des alkyles en $C_1$-$C_{10}$, chlore, méthoxy ou/et éthoxy, un phénylalkyle en $C_7$-$C_{10}$ ou un phénylalkyle en $C_7$-$C_{10}$ substitué sur le reste phényle par des alkyles en $C_1$-$C_{20}$, $X_2$ représente un phényle ou un phényle substitué par 1 à 3 restes, les restes étant choisis parmi le groupe formé des alkyles en $C_1$-$C_4$, chlore, hydroxy, méthoxy, éthoxy et acétylamino, $X_3$ signifie un alkyle en $C_1$-$C_8$ ou un phényle, lorsque n vaut 1, $R_3$ signifie un groupe de formules IIIb à IIIg, $X_0$ est un

atome d'oxygène, $Y_3$ représente un alkyle en $C_4$-$C_{20}$, un alcènyle en $C_3$-$C_{20}$, un phényle, un phényle substitué par des alkyles en $C_1$-$C_4$, chlore, méthoxy ou/et éthoxy, un 2-phényléthènyle, un di-(alkyl en $C_1$-$C_4$)-amino, un (alkyl en $C_1$-$C_8$)-amino, un phénylamino, un benzylamino, un benzène-sulfonamido ou un toluène-sulfonamido, $Y_4$ est un di-(alkyl en $C_1$-$C_4$)-amino, un (alkyle en $C_1$-$C_8$)-amino, un phénylamino ou un benzylamino, $Y_5$ est un alkyle en $C_1$-$C_{20}$, un alkyle en $C_3$-$C_6$ substitué par 1 ou 2 atomes d'oxygène, un cycloalkyle en $C_5$-$C_{12}$, un cycloalkyle en $C_5$-$C_{12}$ substitué par des alkyles en $C_1$-$C_4$, un phényle ou un phényalkyle en $C_7$-$C_{10}$ substitué sur le reste phényle par des alkyles en $C_1$-$C_{20}$, $Y_6$ est un alcoxy en $C_1$-$C_4$, un phénylamino ou un phénylamino substitué sur le groupe phényle par des alkyles en $C_1$-$C_4$, chlore, méthoxy ou/et éthoxy, $Y_7$ représente un phényle ou un tolyle, les restes $Y_8$ signifient, indépendamment l'un de l'autre, des alcoxy en $C_1$-$C_4$ ou des allyloxy, et lorsque n vaut 2, $R_3$ représente un groupe de formule IVa, IVb, IVc ou IVd, $X_0$ a la signification donnée ci-dessus, $Z_1$ signifie une liaison directe, un alkylène en $C_1$-$C_{12}$ ou un phénylène, $Z_2$ est un alkylène en $C_2$-$C_{12}$ ou un 3-oxapentylène, $Z_3$ représente un alkylène en $C_4$-$C_8$ ou un phénylène et Z4 représente un alcoxy en $C_1$-$C_4$ ou un allyloxy.

**17.** Composés selon la revendication 15, dans dans lesquels $X_1$ est un alkyle en $C_1$-$C_{18}$, un alkyle en $C_3$-$C_6$ interrompu par un ou deux atomes d'oxygène ou de soufre ou/et substitué par OH, un allyle, un méthallyle, un oléyle, un cycloalkyle en $C_5$-$C_8$, un phényle, un phényle substitué par des alkyles en $C_1$-$C_{10}$, chlore, méthoxy ou/et éthoxy, un phénylalkyle en $C_7$-$C_{10}$ ou un phénylalkyle en $C_7$-$C_{10}$ substitué sur le reste phényle par des alkyles en $C_8$-$C_{14}$, $X_3$ signifie un méthyle, un éthyle ou un phényle, $Y_3$ est un alkyle en $C_4$-$C_{18}$, un allyle, un méthallyle, un oléyle, un phényle, un phényle substitué par des alkyles en $C_1$-$C_4$,chlore, méthoxy ou/et éthoxy, un 2-phényléthényle ou un di-(alkyl en $C_1$-$C_4$)-amino et $Y_5$ représente un alkyle en $C_1$-$C_{18}$, un cycloalkyle en $C_5$-$C_8$, un phényle ou un phényalkyle en $C_7$-$C_{10}$ substitué sur le reste phényle par des alkyles en $C_8$-$C_{14}$.

**18.** Composés 2-méthyl-3-méthoxycarbonyl-4-éthoxycarbonyl-aminopyrrole, 2-méthyl-3-méthoxycarbonyl-4-benzoylamino-pyrrole, 2-méthyl-3-méthoxycarbonyl-4-(2',4'-diallyloxy-1',3'-5'-triazine-6'-yl)-aminopyrrole, 1,4-bis-[(2'-méthyl-3'-méthoxycarbonylpyrrol-4'-yl)-carbamoyl]-butane ou 1,6-bis-[(2'-méthyl-3'-méthoxycarbonylpyrrol-4'-yl)-carbamoyloxy]-hexane selon la revendication 15.